# EUROPEAN PATENT SPECIFICATION

(11) **EP 3 964 494 B1**
(45) Date of publication and mention of the grant of the patent: **14.01.2026**
(21) Application number: 19927473.9
(22) Date of filing: 20.11.2019
(51) Int. Cl.: C07C 67/343, C07C 69/716, C07C 45/72, C07C 49/258, C07C 49/86, C07D 307/33

(54) **METHOD FOR SYNTHESIZING CHIRAL 2-HYDROXY-1,4-DICARBONYL COMPOUND AND PANTOLACTONE**
VERFAHREN ZUR HERSTELLUNG VON CHIRALEN 2-HYDROXY-1,4-DICARBONYLVERBINDUNGEN UND PANTOLACTON
PROCÉDÉ DE SYNTHÈSE D'UN COMPOSÉ 2-HYDROXY-1,4-DICARBONYLE CHIRAL ET DE PANTOLACTONE

(30) Priority: 29.04.2019 CN 201910352646
(43) Date of publication of application: 09.03.2022
(73) Proprietor: Lanzhou University, Lanzhou, Gansu 730000 (CN); Hangzhou Xinfu Science & Technology Co., Ltd., Hangzhou, Zhejiang 311305 (CN)
(72) Inventor: DA, Chaoshan, Lanzhou, Gansu 730000 (CN); DU, Zhihong, Lanzhou, Gansu 730000 (CN); TAO, Baoxiu, Lanzhou, Gansu 730000 (CN); QIN, Wenjuan, Lanzhou, Gansu 730000 (CN); YUAN, Meng, Lanzhou, Gansu 730000 (CN); BAI, Yanbing, Hangzhou, Zhejiang 311305 (CN); LIN, Hang, Hangzhou, Zhejiang 311305 (CN); ZHANG, Lianchun, Hangzhou, Zhejiang 311305 (CN); YIN, Hanghua, Hangzhou, Zhejiang 311305 (CN); JIANG, Weilin, Hangzhou, Zhejiang 311305 (CN); YU, Jianxin, Hangzhou, Zhejiang 311305 (CN); LIU, Xueyu, Hangzhou, Zhejiang 311305 (CN); JIN, Wenjiu, Hangzhou, Zhejiang 311305 (CN)
(74) Representative: ABG Intellectual Property Law, S.L.
(86) International application number: PCT/CN2019/119629
(87) International publication number: WO 2020/220651

(56) References cited:
- CN-A- 102 985 400
- TAICHI KANO ET AL: "A Designer Axially Chiral Amino Sulfonamide as an Efficient Organocatalyst for Direct Asymmetric anti-Selective Mannich Reactions and syn-Selective Cross-Aldol Reactions", CHEMISTRY - A EUROPEAN JOURNAL, JOHN WILEY & SONS, INC, DE, vol. 15, no. 27, 28 May 2009 (2009-05-28), pages 6678 - 6687, XP071829227, ISSN: 0947-6539, DOI: 10.1002/CHEM.200900267
- MARCEL HEIDLINDEMANN, ET AL.: "Chemoenzymatic Synthesis of Vitamin B5-Intermediate (R)-Pantolactone via Combined Asymmetric Organo- and Biocatalysis", THE JOURNAL OF ORGANIC CHEMISTRY, vol. 80, 28 February 2015 (2015-02-28), pages 3387 - 3396, XP055495038, DOI: 20200211150110Y
- MARCEL HEIDLINDEMANN, ET AL.: "Chemoenzymatic Synthesis of Vitamin B5-Intermediate (R)-Pantolactone via Combined Asymmetric Organo- and Biocatalysis", THE JOURNAL OF ORGANIC CHEMISTRY, vol. 80, 28 February 2015 (2015-02-28), pages 3387 - 3396, XP055495038, DOI: 20200211132740A
- WU, FENGCHUN ET AL.: "N-Primary-Amine-Terminal β-Turn Tetrapeptides as Organocatalysts for Highly Enantioselective Aldol Reaction", THE JOURNAL OF ORGANIC CHEMISTRY, vol. 74, 21 May 2009 (2009-05-21), pages 4812 - 4818, XP055750716, DOI: 20200211133014Y
- WANG, JINJIN ET AL.: "Progress in the Asymmetric Synthesis of Chiral Pantolactone", INDUSTRIAL CATALYSIS, vol. 22, no. 5, 31 May 2014 (2014-05-31), pages 335 - 340, XP055861760
- LAM YU-HONG, ET AL.: "Stereoselectivities of Histidine-Catalyzed Asymmetric Aldol Additions and Contrasts with Proline Catalysis: A Quantum Mechanical Analysis", JOURNAL OF THE AMERICAN CHEMICAL SOCIETY, vol. 134, 31 March 2012 (2012-03-31), pages 6286 - 6295, XP055495037, DOI: 20200211132521A

## Description

This application claims the priority to Chinese Patent Application No. 201910352646.9, titled "METHOD FOR SYNTHESIZING CHIRAL 2-HYDROXY-1,4-DICARBONYL COMPOUNDS AND PANTOLACTONE", filed on April 29, 2019 with the China National Intellectual Property Administration.

### FIELD

The present disclosure relates to the field of asymmetric organic synthesis, and specifically relates to synthesizing chiral 2-hydroxy-1,4-dicarbonyl compounds by an asymmetric aldol reaction between aliphatic aldehydes and glyoxylates or between aliphatic aldehydes and acyl formaldehyde monohydrates by using tetrapeptides or their enantiomers as chiral catalysts, and the application of the synthesized product.

### BACKGROUND

Optically active compounds are ubiquitous in nature and play an irreplaceable role in the life activities of cells and organisms. Many drugs are optically active compounds, for which chirality is an important parameter. The most economical and green way to synthesize a chiral compound is to catalyze an asymmetric reaction with a chiral catalyst.

Currently, asymmetric catalysts mainly include three types, namely, chiral ligand-metal complex catalysts, small organic molecule catalysts and biological enzymes. Bioenzyme catalysis has always been a research hotspot in asymmetric catalysis owing to its green and cost-effective properties, but it has not been quickly applied because enzymes are of structural complexity, easy inactivation, high specificity and other limitations of enzyme proteins. Peptides are a new type of organocatalysts designed and synthesized according to the enzyme catalytic mechanism, and have many advantages. They are structurally simple and stable, easy to be synthesized, tolerant to wide scopes of substrates, applicable to many types of reactions, and usable under mild reaction conditions. They are excellent mimics of natural enzymes, and are the key research direction of chemical biology.

It has been reported that synthetic short peptides have been widely used to catalyze various asymmetric reactions such as aldol and Michael so as to synthesize a variety of optically active compounds [Davie, E. A. C.; Mennen, S. M.; Xu, Y.; Miller, S. J. Chem. Rev. 2007, 107, 5759-5812]. Among them, the asymmetric aldol reaction is the most reported one. In the field of synthesis of chiral compounds, it is always a hot spot to discover new and efficient short peptide catalysts.

The optically active 2-hydroxy-3-methyl-3-formyl butyric acid ester produced by the asymmetric aldol reaction between isobutyraldehyde and glyoxylate is a direct precursor compound for synthesizing optically active pantolactone, showing important application value. Currently, reported chiral catalysts for catalyzing such asymmetric aldol reaction are mainly natural or unnatural amino acids. The result of this aldol reaction catalyzed by proline and its derivatives was the first to be reported, but the highest ee value was only 42% [Zhong, G.; Fan, J.; Barbas, C. F. Tetrahedron Lett. 2004, 45, 5681-5684]. After that, it was found that other amino acids instead of proline can also catalyze this reaction, and its ee value has been increased to a certain degree, e.g., an ee value of 65% by use of 10 mol% of the catalytic amount of natural histidine as the chiral organocatalyst [Markert, M.; Scheffler, U.; Mahrwald, R. J. Am. Chem. Soc. 2009, 131, 16642-16643]. Subsequently, it was found that using 50 mol% of natural isoleucine to promote this reaction in dimethyl sulfoxide as the solvent can obtain a maximum ee value of 77% and a maximum yield of 81% [Rohr, K.; Mahrwald, R. Org. Lett. 2012, 14, 2180-2183]. After that, it was found that the ee value of the reaction can be increased from 65% to 79% with a conversion rate of 79% by optimizing the reaction conditions when similarly using 10 mol% of histidine as the catalyst [Heidlindemann, M.; Hammel, M.; Scheffler, U.; Mahrwald, R.; Hummel, W.; Berkessel, A.; Gröger, H. J. Org. Chem. 2015, 80, 3387-3396]. As recited above, the ee value in all these studies does not exceed 80%, which is far from enough to synthesize applicable pantolactone with high optical activity. Therefore, it is urgent and also challenging to find a new and efficient chiral catalyst for this reaction.

Similarly, the asymmetric aldol reaction between aliphatic aldehydes and acyl formaldehydes (in a form of monohydrate) also can synthesize 2-hydroxy-1,4-dicarbonyl compounds, whose structures are the frameworks of many bioactive compounds and also can be easily converted into a lot of compounds with multiple chiral centers containing a plurality of organic functional groups, which is of great significance in the synthesis of chiral compounds and drugs. The study on the method of asymmetric aldol reaction between aliphatic aldehydes and acyl formaldehyde monohydrates has not been systematically reported, and only can be occasionally seen in some publications [Kano, T.; Maruoka K. Angew. Chem. Int. Ed. 2007, 46, 1738-1740]; [Kano, T.; Maruoka, K. Chem. Eur. J. 2009, 15, 6678-6687]; [Yan X.; Feng, X. M. Synlett. 2008, 73-76], but in these publications they all used small molecule secondary amines as catalysts. Therefore, it is urgent needed to develop the other kind of catalysts and methods for catalyzing such reactions.

### SUMMARY

As regards to the deficiencies in the existing technology above, the technical problem to be solved by the present disclosure is to provide a method for synthesizing chiral 2-hydroxy-1,4-dicarbonyl compounds and pantolactone. The method provided by the present disclosure not only affords high reaction yield, but also achieves significant chiral selectivity with excellent ee value.

The present disclosure provides a method for synthesizing a chiral 2-hydroxy-1,4-dicarbonyl compounds by using tetrapeptide or its enantiomer as a chiral catalyst to catalyze an asymmetric aldol reaction between aliphatic aldehydes and glyoxylates or between aliphatic aldehydes and acyl formaldehyde monohydrates, and further provides a method for synthesizing pantolactone and its analogues with high optical activity.

The present disclosure provides a method for synthesizing chiral 2-hydroxy-1,4-dicarbonyl compounds by an asymmetric aldol reaction between aliphatic aldehydes and glyoxylates or between aliphatic aldehydes and acyl formaldehyde monohydrates catalyzed by using tetrapeptide **TP** or its enantiomer *ent-***TP** as a chiral catalyst, wherein it is an asymmetric catalytic reaction having the chemical reaction equation as shown in Formula 1 or 2: 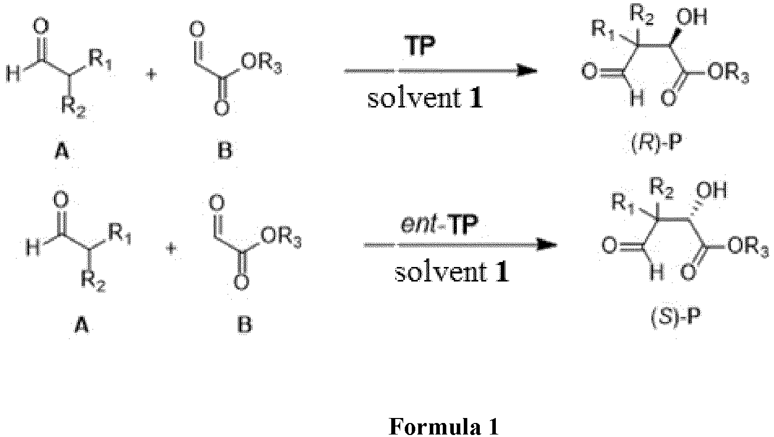 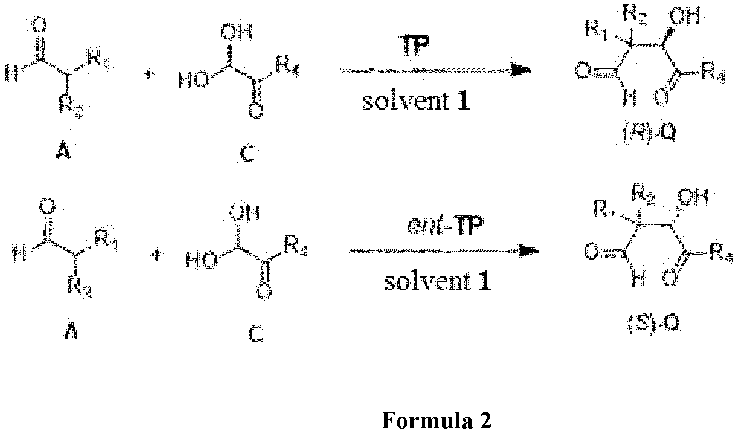 wherein the synthetic process comprises adding aliphatic aldehyde **A,** glyoxylate **B,** and tetrapeptide **TP** as shown in Formula 1 into a reaction vessel containing solvent **1** and stirring to obtain a product (*R*)-**P** in R configuration, or adding aliphatic aldehyde **A,** glyoxylate **B,** and tetrapeptide *ent-***TP** as shown in Formula 1 into a reaction vessel containing solvent **1** and stirring to obtain a product (*S*)-**P** in S configuration; or adding aliphatic aldehyde **A,** acyl formaldehyde monohydrate **C,** and tetrapeptide **TP** as shown in Formula 2 into a reaction vessel containing solvent **1** and stirring to obtain a product (*R*)-**Q** in R configuration, or adding aliphatic aldehyde **A,** acyl formaldehyde monohydrate **C,** and tetrapeptide *ent-***TP** as shown in Formula 2 into a reaction vessel containing solvent **1** and stirring to obtain a product (*S*)-**Q** in S configuration; wherein tetrapeptide **TP** or its enantiomer *ent-***TP** has structure as shown in Formula 3, 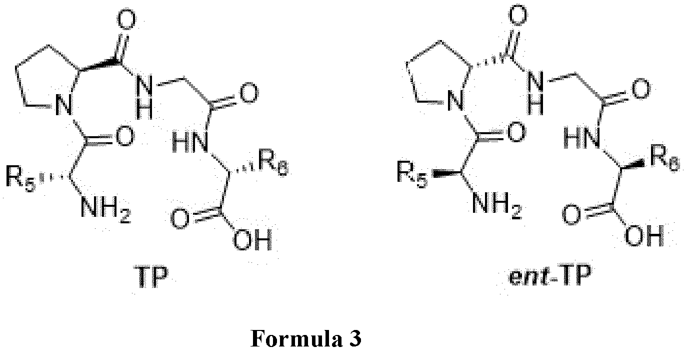 wherein R₁, R₂ are C₁-C₁₀ straight-chain alkyl, branched-chain alkyl or cycloalkyl; R₃ is any one of C₁-C₄ straight-chain alkyl, branched-chain alkyl or benzyl; R₄ is any one of aryl, heteroaryl, arylethyl or arylvinyl; R₅, R₆ are any one of C₁-C₆ straight-chain alkyl, branched-chain alkyl, cyclohexyl, phenyl or benzyl; and solvent **1** is any one of n-hexane, dichloromethane, chloroform, dichloroethane, diethyl ether, tetrahydrofuran, methyltetrahydrofuran, ethylene glycol dimethyl ether, dioxane, ethyl acetate, methyl acetate, ethyl formate, methyl formate, methyl *tert*-butyl ether, acetonitrile, propionitrile, butyronitrile, toluene, xylene, methanol, ethanol, isopropanol, or *n*-butanol, or a mixture thereof.

Preferably, the present disclosure provides a method for synthesizing a chiral 2-hydroxy-1,4-dicarbonyl compound by an asymmetric aldol reaction between aliphatic aldehydes and glyoxylates or between aliphatic aldehydes and acyl formaldehyde monohydrates by using tetrapeptide **TP** or its enantiomer *ent-***TP** as a chiral catalyst, wherein R₁=R₂ is C₁-C₆ straight-chain alkyl, or R₁-R₂ are respectively any one of cycloheptyl, cyclohexyl, cyclopentyl, cyclobutyl or cyclopropyl, R₃ is C₁-C₄ straight-chain alkyl or branched-chain alkyl, R₄ is any one of phenyl, substituted phenyl, naphthyl, heteroaryl, anthryl or arylvinyl, R₅ and R₆ are any one of C₁-C₄ straight-chain alkyl, branched-chain alkyl, cyclohexyl, phenyl or benzyl, and solvent **1** is any one of dichloromethane, 1,2-dichloroethane, chloroform, diethyl ether, tetrahydrofuran, ethyl acetate, acetonitrile, toluene, methanol, or methyl *tert*-butyl ether.

Preferably, the present disclosure provides a method for synthesizing chiral 2-hydroxy-1,4-dicarbonyl compounds by an asymmetric aldol reaction between aliphatic aldehydes and glyoxylates or between aliphatic aldehydes and acyl formaldehyde monohydrates by using tetrapeptide **TP** or its enantiomer *ent-***TP** as a chiral catalyst, wherein the used catalyst **TP** or its enantiomer *ent-***TP** is any one as shown in Formula 4:

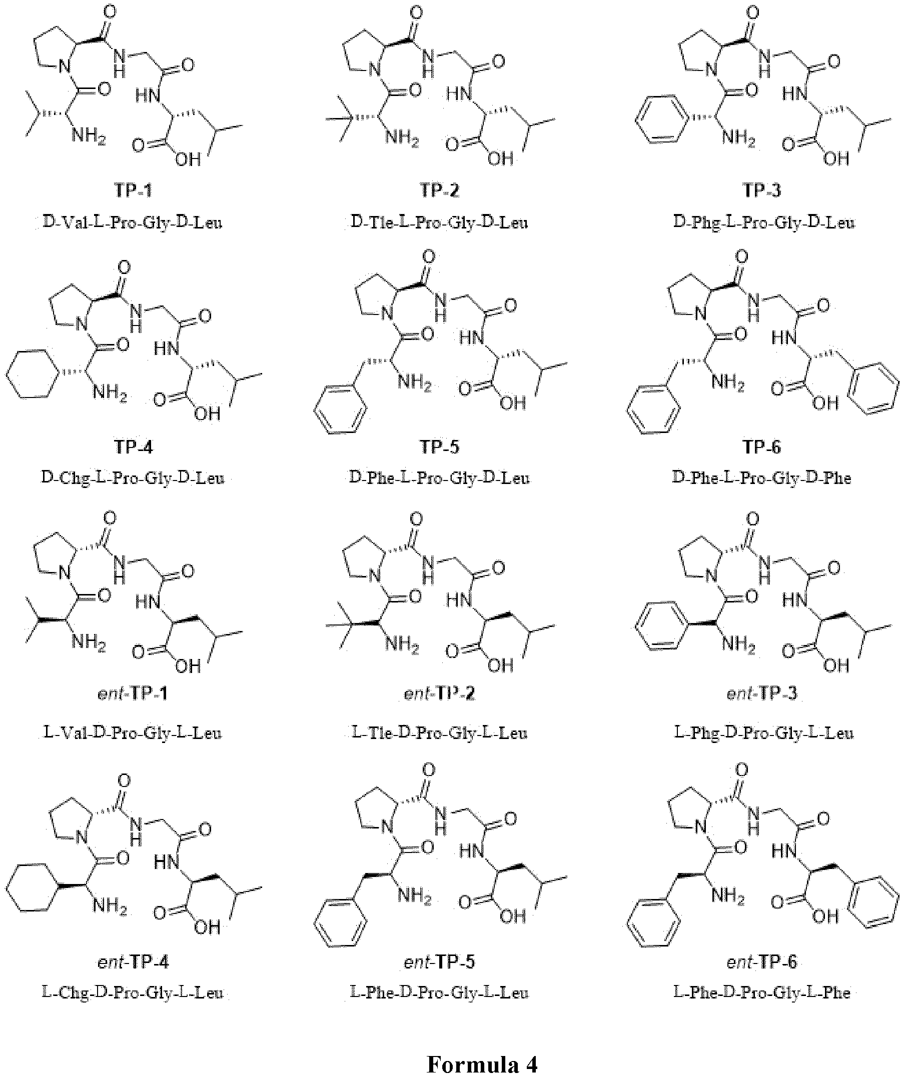

Provided is an application of the product synthesized by the catalytic synthesis method according to the present disclosure for synthesizing chiral pantolactone.

The application of the product synthesized according to the present disclosure for synthesizing chiral pantolactone is shown in Formula 5, comprising adding (*R*)**-P** as shown in Formula 5 into a reaction vessel containing a reducing agent and solvent **2,** stirring and reacting to obtain (*R*)-pantolactone, or adding (*S*)-**P** as shown in Formula 5 into a reaction vessel containing a reducing agent and solvent **2,** stirring and reacting to obtain (*S*)-pantolactone, wherein R₄= Me or Et, and solvent **2** is one or more of dichloromethane, 1,2-dichloroethane, tetrahydrofuran, ethyl acetate, methanol, ethanol, isopropanol, and the reducing agent is one or more of borane, sodium cyanoborohydride, sodium triacetoxyborohydride, sodium borohydride, lithium borohydride, and potassium borohydride,

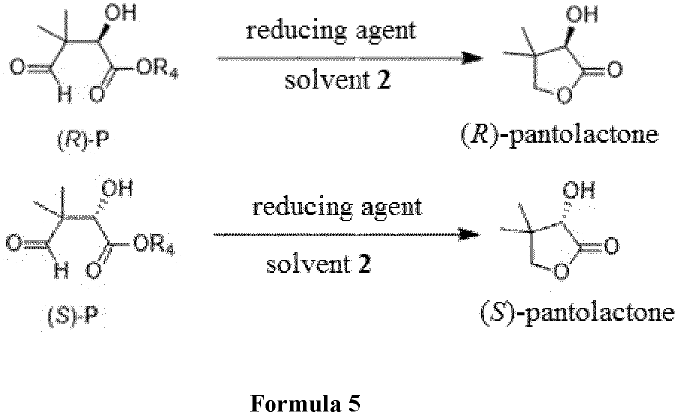

Preferably, provided is an application of the product synthesized according to the present disclosure for synthesizing chiral pantolactone, wherein solvent **2** is either methanol or ethanol, and the reducing agent is any one of sodium cyanoborohydride, sodium triacetoxyborohydride, and sodium borohydride.

It was found from experiments that comparing the method for synthesizing a chiral 2-hydroxy-1,4-dicarbonyl compound and pantolactone provided by the present disclosure with the prior art, the asymmetrically catalyzed aldol reaction provided by the present disclosure undergoes asymmetric catalysis by using tetrapeptide as a chiral catalyst, which enables the reaction to possess so excellent asymmetric catalysis function that the asymmetric aldol reaction between aliphatic aldehydes and glyoxylates or between aliphatic aldehydes and acyl formaldehyde monohydrates can be catalyzed to synthesize optically active 2-hydroxy-1,4-dicarbonyl compounds, and thereby to further synthesize optically active pantolactone. The method is advantaged that it has mild reaction conditions and simple operations, only uses small amount of catalysts, and enables 2-hydroxy-1,4-dicarbonyl compounds to be synthesized with two configurations thereof by using tetrapeptide and its enantiomer, at a high yield up to 99%, and at a high enantioselectivity up to 99% ee, so it has good application potential. Some products synthesized by this method can be used to synthesize optically active pantolactone at a high yield and high enantioselectivity, with ee value reached to 99%.

### DETAILED DESCRIPTION

In order to enable those skilled in the related fields to well understand the technical solutions of this disclosure, this disclosure will be further described in detail below in conjunction with specific embodiments.

The present disclosure provides a method for synthesizing a chiral 2-hydroxy-1,4-dicarbonyl compound, comprising:
reacting a compound represented by Formula **(A)** with a compound represented by Formula **(B)** in the presence of a catalyst with structure represented by Formula **(TP)** or Formula (*ent-***TP**)*,* to obtain a compound represented by Formula (*R*)-**P** or Formula (*S*)-**P**, 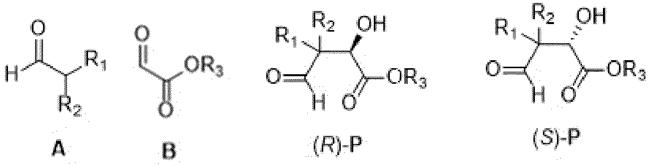 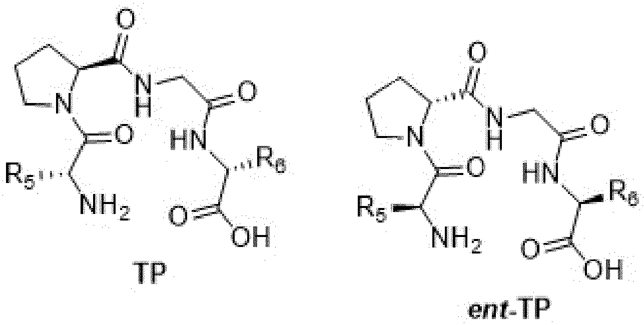
wherein R₁, R₂ are independently selected from straight-chain alkyl or branched-chain alkyl, or R₁ and R₂ together with the carbon to which they are bonded form cycloalkyl, R₃ is selected from any one of C₁-C₄ straight-chain alkyl, branched-chain alkyl or benzyl, and
R₅, R₆ are independently selected from C₁-C₆ straight-chain alkyl, C₃-C₆ branched-chain alkyl, C₅-C₈ cycloalkyl, C₆-C₂₀ aryl or C₇-C₃₀ arylalkyl; or
reacting a compound represented by Formula **(A)** with a compound represented by Formula **(C)** in the presence of a catalyst with structure represented by Formula **(TP)** or Formula (*ent-***TP**)*,* to obtain a compound represented by Formula (*R*)-**Q** or Formula (*S*)-**Q**, 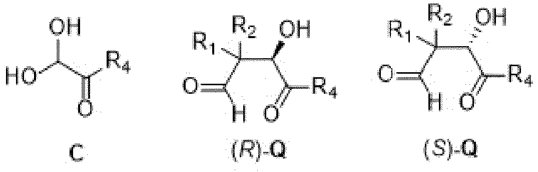
wherein R₄ is selected from any one of aryl, heteroaryl, arylethyl or arylvinyl.

According to the present disclosure, in the present disclosure, a compound represented by Formula **(A)** is reacted with a compound represented by Formula **(B)** in the presence of a catalyst with structure represented by Formula **(TP)** or Formula (ent*-***TP**) to obtain a compound represented by Formula (*R*)-**P** or Formula (*S*)-**P**, wherein in the compound represented by Formula **(A),** R₁, R₂ are independently selected from C₁-C₁₀ straight-chain alkyl or C₃-C₁₀ branched-chain alkyl; or R₁ and R₂ together with the carbon to which they are bonded form C₃-C₈ cycloalkyl, preferably C₁-C₄ straight-chain alkyl, C₃-C₄ branched-chain alkyl, or -R₁-R₂- are selected from C₅-C₇ cycloalkyl, most preferably methyl, ethyl, *n*-propyl, isopropyl, *n*-butyl, isobutyl, *tert*-butyl, *n*-pentyl, isopentyl, or *n*-hexyl; or R₁ and R₂ together with the carbon to which they are bonded form cyclopentyl, cyclohexyl or cycloheptyl; in the compound represented by Formula **(B)** R₃ is selected from C₁-C₄ straight-chain alkyl or C₁-C₄ branched-chain alkyl, preferably methyl, ethyl, *n*-propyl, isopropyl, *n*-butyl, isobutyl or *tert*-butyl; in the catalyst with structure represented by Formula **(TP)** or Formula (*ent-**TP***)*,* R₅, R₆ are preferably independently selected from methyl, ethyl, *n*-propyl, isopropyl, *n*-butyl, isobutyl, *tert*-butyl*, n*-pentyl, isopentyl, *n*-hexyl, cyclopentyl, cyclohexyl, cycloheptyl, phenyl, naphthyl, anthryl, benzyl or phenethyl.

Specifically, the chiral catalyst is shown as follows:

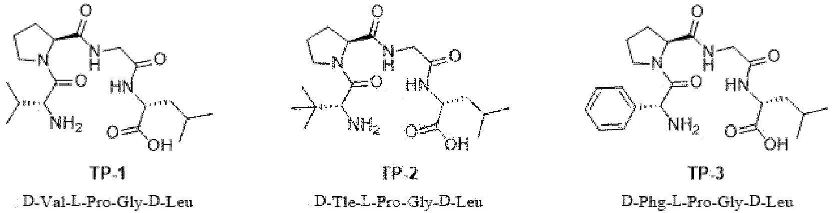

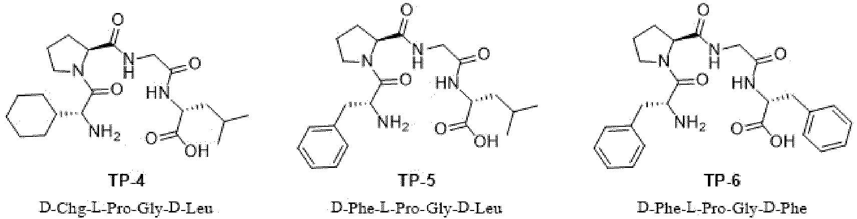

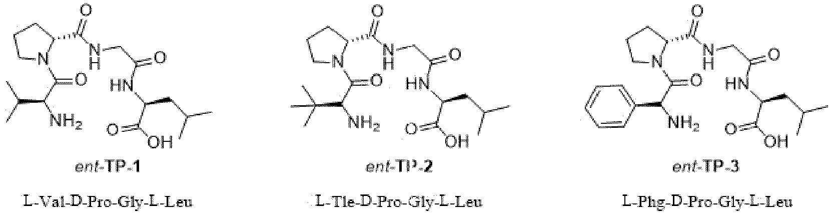

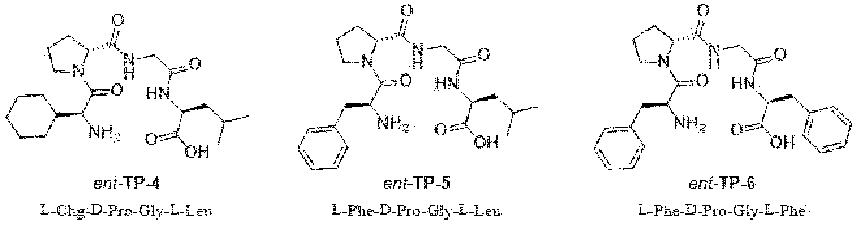

Specifically, the catalytic reaction follows the chemical reaction equation as shown in Formula 1, wherein solvent **1** in the reaction is one or more of *n*-hexane, dichloromethane, chloroform, dichloroethane, diethyl ether, tetrahydrofuran, methyltetrahydrofuran, ethylene glycol dimethyl ether, dioxane, ethyl acetate, methyl acetate, ethyl formate, methyl formate, methyl *tert*-butyl ether, acetonitrile, propionitrile, butyronitrile, toluene, xylene, methanol, ethanol, isopropanol and *n*-butanol, and preferably is any one of dichloromethane, 1,2-dichloroethane, chloroform, diethyl ether, tetrahydrofuran, ethyl acetate, acetonitrile, toluene, methanol, or methyl *tert*-butyl ether.

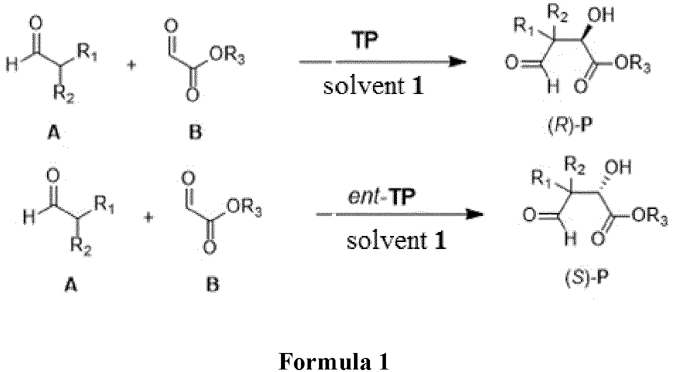

Furthermore, according to the present disclosure, in the present disclosure, a compound represented by Formula **(A)** is reacted with a compound represented by Formula **(C)** in the presence of a catalyst with structure represented by Formula **(TP)** or Formula (***ent-TP***)*,* to obtain a compound represented by Formula (*R*)-**Q** or Formula (*S*)-**Q** (as shown in Formula 2), wherein in the compound represented by Formula **(C),** R₄ is phenyl, substituted phenyl, naphthyl, heteroaryl, anthryl or arylvinyl, and preferably phenyl, substituted phenyl, C₅-C₂₀ aryl containing a heteroatom, anthryl or arylvinyl, wherein the substitution group in the substituted phenyl is preferably C₁-C₁₀ alkyl, halogen, C₁-C₁₀ alkoxy or C₄-C₂₀ heteroaryl; the heteroatom in the aryl containing a heteroatom or heteroaryl is nitrogen, oxygen or sulfur; and solvent **1** in the reaction is one or more of *n*-hexane, dichloromethane, chloroform, dichloroethane, diethyl ether, tetrahydrofuran, methyltetrahydrofuran, ethylene glycol dimethyl ether, dioxane, ethyl acetate, methyl acetate, ethyl formate, methyl formate, methyl *tert*-butyl ether, acetonitrile, propionitrile, butyronitrile, toluene, xylene, methanol, ethanol, isopropanol and *n*-butanol, and is preferably any one of dichloromethane, 1,2-dichloroethane, chloroform, diethyl ether, tetrahydrofuran, ethyl acetate, acetonitrile, toluene, methanol or methyl *tert*-butyl ether.

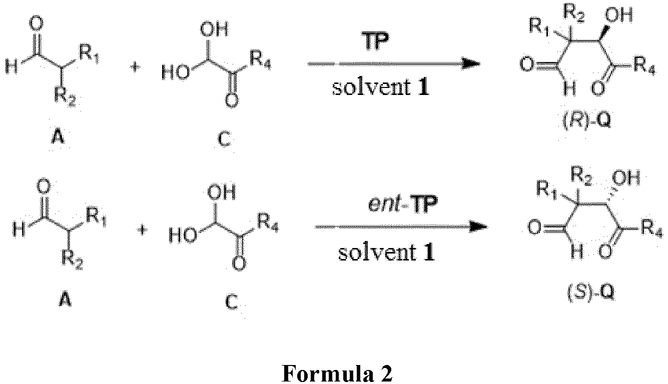

More specifically, the synthesis process of chiral 2-hydroxy-1,4-dicarbonyl compound comprises adding aliphatic aldehyde **A,** glyoxylate **B,** and tetrapeptide **TP** as shown in Formula 1 into a reaction vessel containing solvent **1** and stirring to obtain a product (*R*)-**P** in R configuration, or adding aliphatic aldehyde **A,** glyoxylate **B,** and tetrapeptide *ent-***TP** as shown in Formula 1 into a reaction vessel containing solvent **1** and stirring to obtain a product (*S*)-**P** in S configuration; or adding aliphatic aldehyde **A,** acyl formaldehyde monohydrate **C,** and tetrapeptide **TP** as shown in Formula 2 into a reaction vessel containing solvent **1** and stirring to obtain a product (*R*)-**Q** in R configuration, or adding aliphatic aldehyde **A,** acyl formaldehyde monohydrate **C,** and tetrapeptide *ent-***TP** as shown in Formula 2 into a reaction vessel containing solvent **1** and stirring to obtain a product (*S*)-**Q** in S configuration; wherein tetrapeptide **TP** or its enantiomer *ent-***TP** has structure as shown in Formula 3, 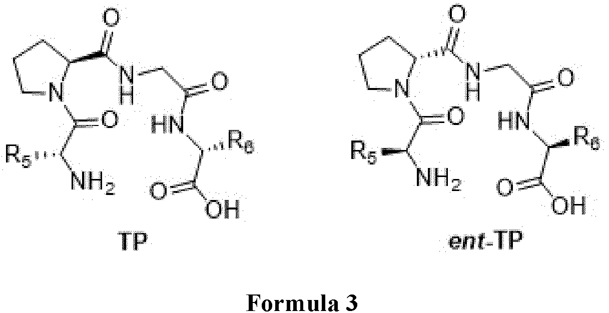 wherein R₁, R₂ are C₁-C₁₀ straight-chain alkyl or branched-chain alkyl, or R₁ and R₂ together with the carbon to which they are bonded form cycloalkyl; R₃ is any one of C₁-C₄ straight-chain alkyl, branched-chain alkyl or benzyl; R₄ is any one of aryl, heteroaryl, arylethyl or arylvinyl; R₅, R₆ are any one of C₁-C₆ straight-chain alkyl, branched-chain alkyl, cyclohexyl, phenyl or benzyl; and solvent **1** is any one of *n*-hexane, dichloromethane, chloroform, dichloroethane, diethyl ether, tetrahydrofuran, methyltetrahydrofuran, ethylene glycol dimethyl ether, dioxane, ethyl acetate, methyl acetate, ethyl formate, methyl formate, methyl ter*t*-butyl ether, acetonitrile, propionitrile, butyronitrile, toluene, xylene, methanol, ethanol, isopropanol, or *n*-butanol, or a mixture thereof.

The present disclosure further provides a method of preparing chiral pantolactone and an analogue thereof, comprising:
preparing the compound of Formula (*R*)-**P** or Formula (*S*)-**P** by using the method according to the present disclosure and performing a reduction reaction of the compound of Formula (*R*)-**P** or Formula (*S*)-**P**, to obtain pantolactone in R or S configuration or an analogue thereof, wherein solvent **2** in the reaction is one or more of dichloromethane, 1,2-dichloroethane, tetrahydrofuran, ethyl acetate, methanol, ethanol and isopropanol, and preferably is one or two of methanol and ethanol; and the reducing agent in the reaction is one or more of borane, sodium cyanoborohydride, sodium triacetoxyborohydride, sodium borohydride, lithium borohydride and potassium borohydride, and preferably is any one of sodium cyanoborohydride, sodium triacetoxyborohydride or sodium borohydride.

More specifically, the preparation method is shown in Formula 5, comprising adding (*R*)-**P** as shown in Formula 5 into a reaction vessel containing a reducing agent and solvent **2,** stirring and reacting to obtain (*R*)-pantolactone, or adding (*S*)-**P** as shown in Formula 5 into a reaction vessel containing a reducing agent and solvent **2,** stirring and reacting to obtain (*S*)-pantolactone; wherein R₄ is Me or Et, and solvent **2** is one or more of dichloromethane, 1,2-dichloroethane, tetrahydrofuran, ethyl acetate, methanol, ethanol and isopropanol; and the reducing agent is one or more of borane, sodium cyanoborohydride, sodium triacetoxyborohydride, sodium borohydride, lithium borohydride and potassium borohydride.

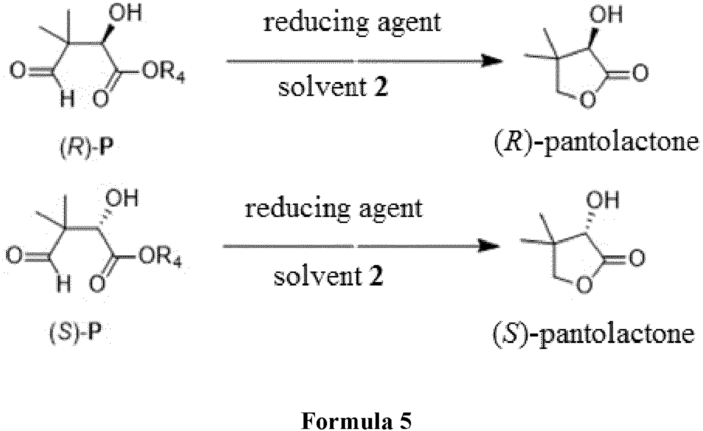

The inventors found in their study on asymmetrically catalyzed aldol reactions that a plurality of tetrapeptide catalysts possess so excellent asymmetric catalysis function that the asymmetric aldol reaction between aliphatic aldehydes and glyoxylates or between aliphatic aldehydes and acyl formaldehyde monohydrates can be catalyzed to synthesize highly optically active 2-hydroxy-1,4-dicarbonyl compounds, and thereby to further synthesize optically active pantolactone. The method is advantaged that it has mild reaction conditions and simple operations, only uses small amount of catalysts, and enables 2-hydroxy-1,4-dicarbonyl compounds to be synthesized with two configurations thereof by using the tetrapeptide and its enantiomer, at a high yield up to 99%, and at a high enantioselectivity up to 99% ee, so it has good application potential. Some products synthesized by this method can be used to synthesize optically active pantolactone at a high yield and high enantioselectivity, with ee value reached to 99%.

The present disclosure has been introduced in detail above. Hereinafter, the application will be further described in detail in conjunction with examples. However, it is necessary to point out that the following specific embodiments are intended to further illustrate this application, but should not be considered to limit the scope of protection of the application. It is possible for those skilled in the related fields to make some non-essential improvement and adjustment to this application based on the content of the application described above.

### Example 1

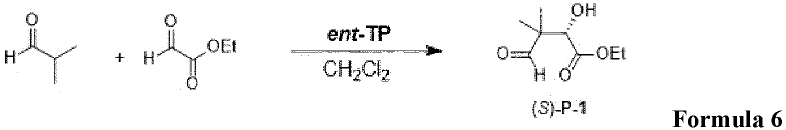

To a 5 mL round-bottomed flask, accurately weighed tetrapeptide catalyst *ent-***TP** (0.025 mmol) and 1.0 mL of dichloromethane were added, isobutyraldehyde (92 µL, 1.0 mmol), and then ethyl glyoxylate (50% toluene solution, 0.1 mL, 0.5 mmol) were added while stirring in an ice-water bath. The reaction mixture was allowed to return to room temperature and stirred. The reaction process was judged through TLC detection using 2,4-dinitrophenylhydrazine stain. After the reaction was completed, the reaction mixture was added with 3-4 drops of saturated ammonium chloride solution, extracted with ethyl acetate (10 mL × 3), washed with small amount of saturated brine, dried with anhydrous sodium sulfate, and concentrated under reduced pressure. The resulting oily residue was purified by column chromatography, to obtain product (*S*)-**P-1**.

**Table 1. Results of the asymmetric aldol reaction between isobutyraldehyde and ethyl glyoxylate catalyzed by using ent-TP-1 - ent-TP-6 as tetrapeptide catalysts**

| Entry | *ent-***TP** (0.025 mmol) | time (d) | yield (%)^{a} | ee (%)^{b} |
|---|---|---|---|---|
| 1 | *ent-***TP-1** | 2 d | 79% | 94 |
| 2 | *ent-***TP-2** | 3 d | 85% | 97 |
| 3 | *ent-***TP-3** | 3 d | 59% | 75 |
| 4 | *ent-***TP-4** | 2 d | 80% | 91 |
| 5 | *ent-***TP-5** | 3 d | 83% | 91 |
| 6 | *ent-***TP-6** | 3 d | 84% | 84 |

| | | | | |
|---|---|---|---|---|
| ^{a}Isolated yield. ^{b} ee values were measured by performing chiral HPLC on benzoic acid ester of product (*S*)-**P-1**. | | | | |

### Example 2

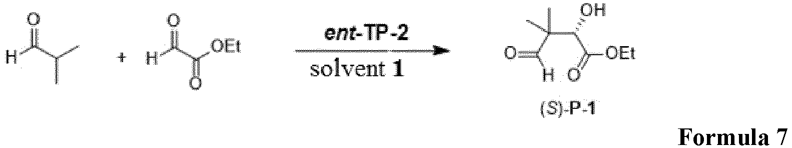

To a 5 mL round-bottomed flask, accurately weighed tetrapeptide catalyst *ent-***TP-2** (0.025 mmol, 10 mg) and 1.0 mL of solvent were added, isobutyraldehyde (92 µL, 1.0 mmol), and then ethyl glyoxylate (50% toluene solution, 0.1 mL, 0.5 mmol) were added while stirring in an ice-water bath. The reaction mixture was allowed to return to room temperature and stirred. The reaction process was judged through TLC detection using 2,4-dinitrophenylhydrazine stain. After the reaction was completed, the reaction mixture was added with 3-4 drops of saturated ammonium chloride solution to quench the reaction, extracted with ethyl acetate (10 mL × 3), washed with small amount of saturated brine, dried with anhydrous sodium sulfate, and concentrated under reduced pressure. The resulting oily residue was purified by column chromatography, to obtain (*S*)-**P-1**.

**Table 2. Effects of solvents on the asymmetric aldol reaction between isobutyraldehyde and ethyl glyoxylate catalyzed by tetrapeptide ent-TP-2**

| entry | solvent | time | yield %^{a} | ee %^{b} |
|---|---|---|---|---|
| 1 | CH₂Cl₂ | 3 d | 85 | 97 |
| 2 | PhMe | 2 d | 87 | 95 |
| 3 | ClCH₂CH₂Cl | 3 d | 86 | 98 |
| 4 | CHCl₃ | 3 d | 57 | 98 |
| 5 | MeOH | 2 d | 91 | 86 |
| 6 | Et₂O | 3 d | 80 | 95 |
| 7 | THF | 2 d | 90 | 98 |
| 8 | MeCN | 1.5 d | 98 | 99 |
| 9 | MeOt-Bu | 1d | 84 | 91 |

| | | | | |
|---|---|---|---|---|
| ^{a}Isolated yield. ^{b} ee values were measured by performing chiral HPLC on benzoic acid ester of product (*S*)-**P-1**. | | | | |

### Example 3

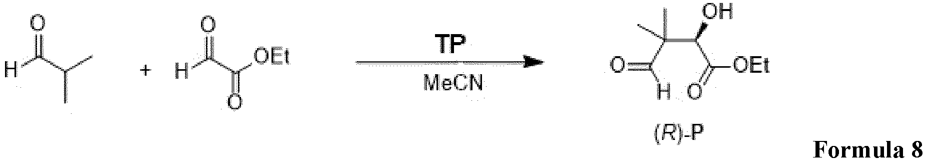

To a 5 mL round-bottomed flask, accurately weighed tetrapeptide catalyst **TP** (0.025 mmol) and 1.0 mL of acetonitrile were added, isobutyraldehyde (92 µL, 1.0 mmol), and then ethyl glyoxylate (50% toluene solution, 0.1 mL, 0.5 mmol) were added while stirring in an ice-water bath. The reaction mixture was allowed to return to room temperature and stirred. The reaction process was judged through TLC detection using 2,4-dinitrophenylhydrazine stain. After the reaction was completed, the reaction mixture was added with 3-4 drops of saturated ammonium chloride solution, extracted with ethyl acetate (10 mL × 3), washed with small amount of saturated brine, dried with anhydrous sodium sulfate, and concentrated under reduced pressure. The resulting oily residue was purified by column chromatography, to obtain reaction product (*R*)-**P-1**.

**Table 3. Asymmetric aldol reaction between isobutyraldehyde and ethyl glyoxylate catalyzed by tetrapeptide TP-1 - TP-6**

| Entry | **TP** (0.025 mmol) | time (d) | yield (%)^{a} | ee (%)^{b} |
|---|---|---|---|---|
| 1 | **TP-1** | 2 d | 89 | -96 |
| 2 | **TP-2** | 3 d | 97 | -99 |
| 3 | **TP-3** | 3 d | 93 | -96 |
| 4 | **TP-4** | 2 d | 88 | -91 |
| 5 | **TP-5** | 3 d | 95 | -98 |
| 6 | **TP-6** | 3 d | 87 | -91 |

| | | | | |
|---|---|---|---|---|
| ^{a}Isolated yield. ^{b} ee values were measured by performing chiral HPLC on benzoic acid ester of product (*R*)-**P-1**. | | | | |

### Example 4

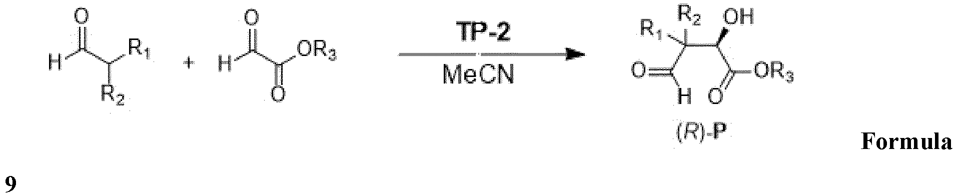

To a 5 mL round-bottomed flask, accurately weighed tetrapeptide catalyst **TP-2** (0.025 mmol, 10 mg) and 1.0 mL of acetonitrile were added, aliphatic aldehyde (1.0 mmol), and then glyoxylate (0.5 mmol) were added while stirring in an ice-water bath. The reaction mixture was allowed to return to room temperature and stirred. The reaction process was judged through TLC detection using 2,4-dinitrophenylhydrazine stain. After the reaction was completed, the reaction mixture was added with 3-4 drops of saturated ammonium chloride solution to quench the reaction, extracted with ethyl acetate (10 mL × 3), washed with small amount of saturated brine, dried with anhydrous sodium sulfate, and concentrated under reduced pressure. The resulting oily residue was purified by column chromatography, to obtain product.

**Table 4. Asymmetric aldol reaction between aliphatic aldehyde and glyoxylate catalyzed by tetrapeptide TP-2^{a}**

| | | |
|---|---|---|
| 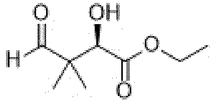 (*R*)-**P-1**, 97% yield, 99% ee | 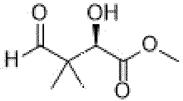 (*R*)-**P-2**, 92% yield, 99% ee | 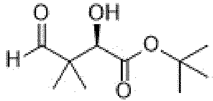 (*R*)-**P-3**, 89% yield, 99% ee |
| 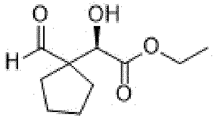 (*R*)-**P-4**, 91% yield, 99% ee | 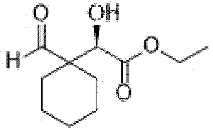 (*R*)-**P-5,** 93% yield, 97% ee | 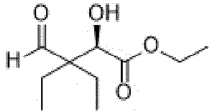 (*R*)-**P-6**, 85% yield, 99% ee |

| | | |
|---|---|---|
| *^{a}* Isolated yield. ee values were measured by performing chiral HPLC on benzoic acid ester of product (*R*)-**P**. | | |

### Example 5

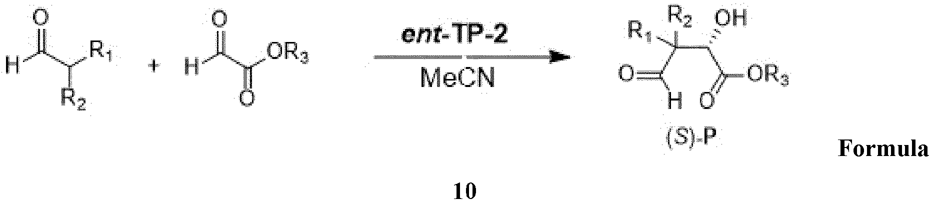

To a 5 mL round-bottomed flask, accurately weighed tetrapeptide catalyst *ent-***TP-2** (0.025 mmol, 10 mg) and 1.0 mL of acetonitrile were added, aliphatic aldehyde (1.0 mmol), and then glyoxylate (0.5 mmol) were added while stirring in an ice-water bath. The reaction mixture was allowed to return to room temperature and stirred. The reaction process was judged through TLC detection using 2,4-dinitrophenylhydrazine stain. After the reaction was completed, the reaction mixture was added with 3-4 drops of saturated ammonium chloride solution to quench the reaction, extracted with ethyl acetate (10 mL × 3), washed with small amount of saturated brine, dried with anhydrous sodium sulfate, and concentrated under reduced pressure. The resulting oily residue was purified by column chromatography, to obtain product.

**Table 5. Asymmetric aldol reaction between aliphatic aldehyde and glyoxylate catalyzed by tetrapeptide ent-TP-2^{a}**

| | | |
|---|---|---|
| 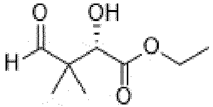 (*S*)-**P-11**, 98% yield, 99% ee | 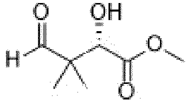 (*S*)-**P-2**, 90% yield, 99% ee | 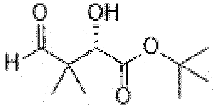 (*S*)-**P-3**, 90% yield, 98% ee |
| 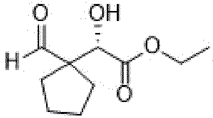 (*S*)-**P-4**, 90% yield, 99% ee | 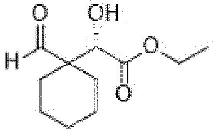 (*S*)-**P-5**, 95%, yield, 97% ee | 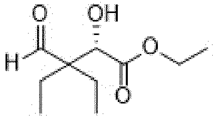 (*S*)-**P-6**, 78% yield, 99% ee |

| | | |
|---|---|---|
| ^{a} Isolated yield, ee values were measured by performing chiral HPLC on benzoic acid ester of product (*S*)-**P**. | | |

### Example 6

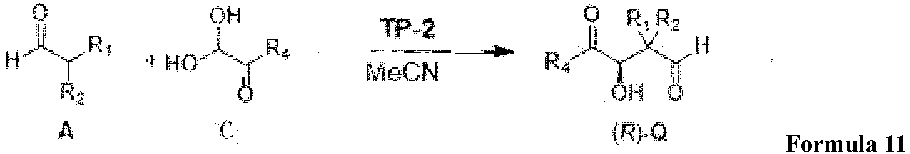

To a 5 mL round-bottomed flask, accurately weighed tetrapeptide catalyst **TP-2** (0.025 mmol, 10 mg) and 1.0 mL of acetonitrile were added, aliphatic aldehyde **A** (1.0 mmol), and then acyl formaldehyde monohydrate **C** (0.5 mmol) were added while stirring in an ice-water bath. The reaction mixture was allowed to return to room temperature and stirred. The reaction process was judged through TLC detection using 2,4-dinitrophenylhydrazine stain. After the reaction was completed, the reaction mixture was added with 3-4 drops of saturated ammonium chloride solution, extracted with ethyl acetate (10 mL × 3), washed with small amount of saturated brine, dried with anhydrous sodium sulfate, and concentrated under reduced pressure. The resulting oily residue was purified by column chromatography, to obtain product.

**Table 6. Asymmetric Aldol reaction between aliphatic aldehyde and acyl formaldehyde monohydrate catalyzed by TP-2.^{a}**

| | | | |
|---|---|---|---|
| 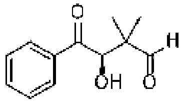 (*R*)-**Q-1,** y=95%, ee=98% | 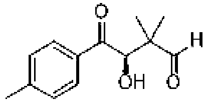 (*R*)-**Q-2**, y=94%, ee=97% | 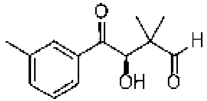 (*R*)-**Q-3,** y=92%, ee=97% | 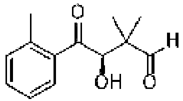 (*R*)-**Q-4**, y=90%, ee=98% |
| 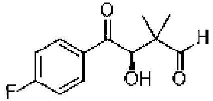 (*R*)-**Q-5,** y=95%, ee=96% | 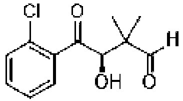 (*R*)-**Q-6.** y=93%, ee=99% | 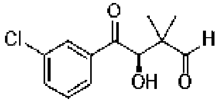 (*R*)-**Q.7**, y=93%. ee=96% | 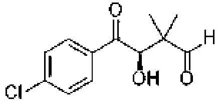 (*R*)-**Q.8**, y=96%, ee=96% |
| 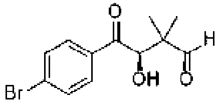 (*R*)-**Q-9**, y=95%, ee=96% | 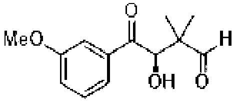 (*R*)-**Q-10**, y=92%, ee=97% | 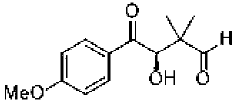 (*R*)-**Q-11**, y=98%, ee=98% | 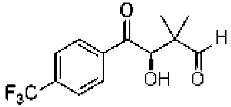 (*R*)-**Q-12,** y=90%, ee=98% |
| 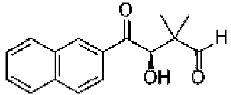 (*R*)-**Q-13**, y=92%, ee=98% | 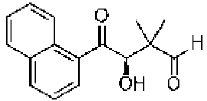 (*R*)-**Q-14**, y=96%, ee=99% | 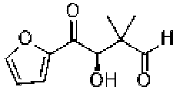 (*R*)-**Q-15**, y=86%, ee=98% | 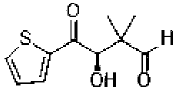 (*R*)-**Q-16**, y=83%, ee=96% |
| 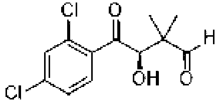 (*R*)-**Q-17**, y=99%, ee=98% | 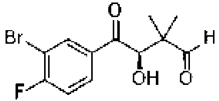 (*R*)-**Q-18,** y=98%, ee=99% | 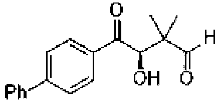 (*R*)-**Q-19**, y=96%, ee=99% | 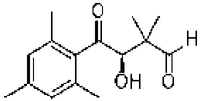 (*R*)-**Q-20**, y=97%, ee=95% |
| 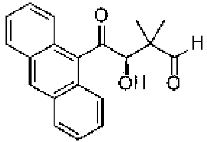 (*R*)-**Q-21**, y=95%, ee=97% | 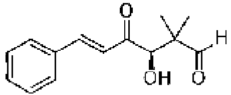 (*R*)-**Q-22;** y=96%, ee=95% | 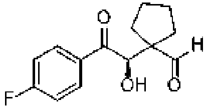 (*R*)-**Q-23**, y=95%, ee=95% | 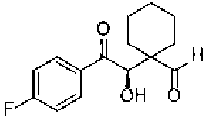 (*R*)-**Q-24**, y=95%, ee=94% |

| | | | |
|---|---|---|---|
| *^{a}*Isolated yield. ee values were measured by performing chiral HPLC after product Q and neopentyl glycol formed acetal. | | | |

### Example 7

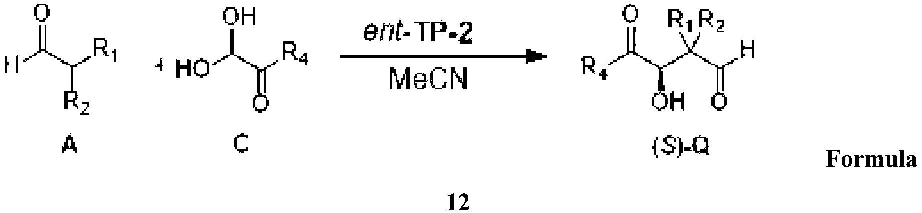

To a 5 mL round-bottomed flask, accurately weighed tetrapeptide catalyst *ent-***TP-2** (0.025 mmol, 10 mg) and 1.0 mL of acetonitrile were added, aliphatic aldehyde **A** (1.0 mmol), and then acyl formaldehyde monohydrate **C** (0.5 mmol) were added while stirring in an ice-water bath. The reaction mixture was allowed to return to room temperature and stirred. The reaction process was judged through TLC detection using 2,4-dinitrophenylhydrazine stain. After the reaction was completed, the reaction mixture was added with 3-4 drops of saturated ammonium chloride solution, extracted with ethyl acetate (10 mL × 3), washed with small amount of saturated brine, dried with anhydrous sodium sulfate, and concentrated under reduced pressure. The resulting oily residue was purified by column chromatography, to obtain product.

**Table 7. Asymmetric Aldol reaction between aliphatic aldehyde and acyl formaldehyde monohydrate catalyzed by ent-TP-2.^{a}**

| | | | |
|---|---|---|---|
| 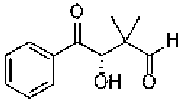 (*S*)-**Q-1**, y=95%, ee=99% | 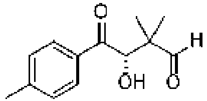 (*S*)-**Q-2_{,}** y=95%, ee=99% | 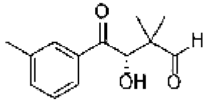 (*S*)-**Q-3**, y=90%, ee=99% | 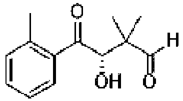 (*S*)-**Q-4**, y=90%, ee=99% |
| 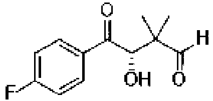 (*S*)-**Q-5**, y=95%, ee=99% | 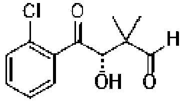 (*S*)-**Q-6**, y=93%, ee=99% | 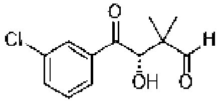 (*S*)-**Q-7**, y=95%, ee=99% | 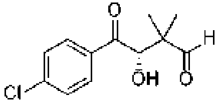 (*S*)-**Q-8**, y=95%, ee=99% |
| 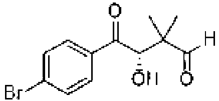 (*S*)-**Q-9**, y=93%. ee=99% | 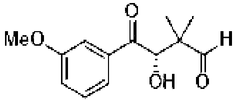 (*S*)-**Q-10**, y=95%, ee=99% | 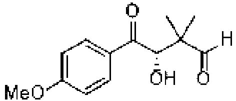 (*S*)-**Q-11**, y=97%, ee=99% | 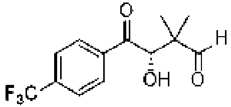 (*S*)-**Q-12**, y=88%, ee=99% |
| 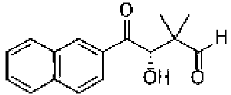 (*S*)-**Q-13**, y=95%, ee=99% | 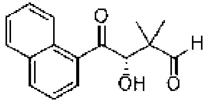 (*S*)-**Q-14**, y=97%, ee=99% | 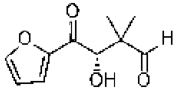 (*S*)-**Q-15,** y=87%, ee=99% | 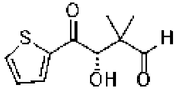 (*S*)-**Q-16,** y=85%, ee=99% |
| 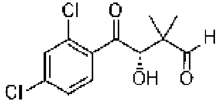 (*S*)-**Q-17**, y=99%, ee=99% | 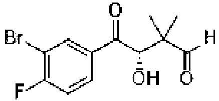 (*S*)-**Q-18**, y=98%, ee=99% | 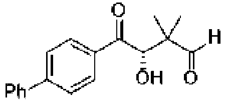 (*S*)-**Q-19,** y=94%, ee=99% | 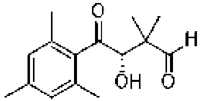 (*S*)-**Q-20**, y=98%, ee=99% |
| 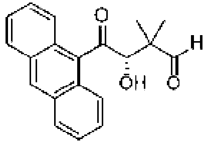 (*S*)-**Q-21**, y=93%, ee=95% | 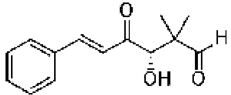 (*S*)-**Q-22**, y=90%, ee=96% | 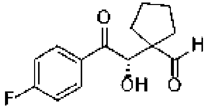 (*S*)-**Q-23**, y=95%, ee=99% | 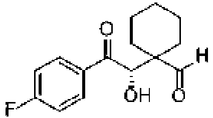 (*S*)-**Q-24**, y=96%, ee=99% |

| | | | |
|---|---|---|---|
| *^{a}* Isolated yield. ee values were measured by performing chiral HPLC after product Q and neopentyl glycol formed acetal. | | | |

### Example 8

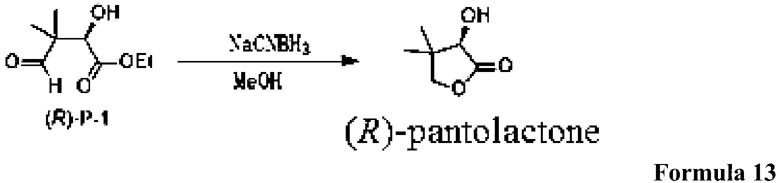

(*R*)**-P-1** (317.8 mg, 1.8 mmol) having an optical purity of 99% ee was dissolved in 5 mL of methanol. The reaction flask was placed in an ice-water bath, and sodium cyanoborohydride (137.6 mg, 2.19 mmol) was slowly added into the reaction solution with stirring in batches, and the stirring was continued until the completion of the reaction. The reaction was quenched by slow addition of 5% dilute hydrochloric acid. The reaction mixture was then extracted three times with ethyl acetate. The ethyl acetate phases were combined, washed with a small amount of saturated brine, dried with anhydrous sodium sulfate, concentrated under reduced pressure, and then quickly purified by silica gel column chromatography, to obtain 218 mg (R)-pantolactone with a yield of 93%, and 99% ee.

### Example 9

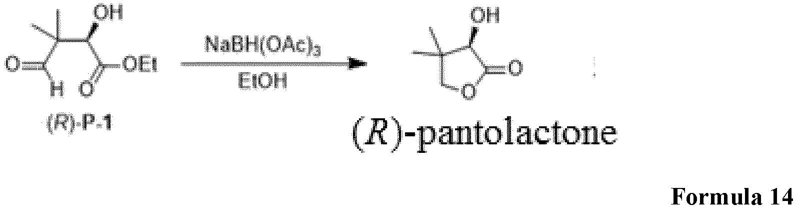

(*R*)-**P-1** (174.3 mg, 1.0 mmol) having an optical purity of 99% ee was dissolved in 5 mL of ethanol. The reaction flask was placed in an ice-water bath, and sodium triacetoxyborohydride (254.4 mg, 1.2 mmol) was slowly added into the reaction solution with stirring in batches, and the stirring was continued until the completion of reaction. The reaction was quenched by slow addition of 5% dilute hydrochloric acid. The reaction mixture was then extracted three times with ethyl acetate. The ethyl acetate phases were combined, washed with a small amount of saturated brine, dried with anhydrous sodium sulfate, concentrated under reduced pressure, and then quickly purified by silica gel column chromatography, to obtain 117 mg (R)-pantolactone with a yield of 90%, and 99% ee.

### Example 10

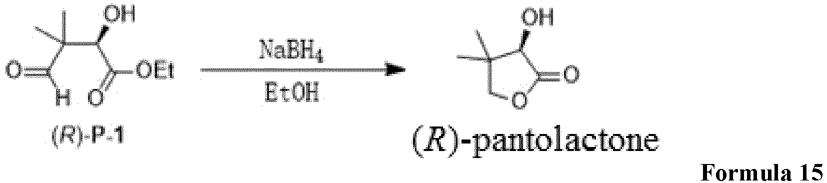

(*R*)-**P-1** (174.3 mg, 1.0 mmol) was dissolved in 5 mL of ethanol. The reaction flask was placed in an ice-water bath, and sodium borohydride (45.4 mg, 1.2 mmol) was slowly added into the reaction solution with stirring in batches, and the stirring was continued until the completion of reaction. The reaction was quenched by slow addition of 5% dilute hydrochloric acid. The reaction mixture was then extracted three times with ethyl acetate. The ethyl acetate phases were combined, washed with a small amount of saturated brine, dried with anhydrous sodium sulfate, concentrated under reduced pressure, and then quickly purified by silica gel column chromatography, to obtain 115 mg (R)-pantolactone with a yield of 88%, and 99% ee.

### Example 11

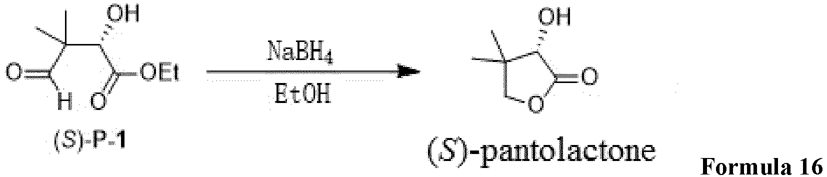

(*S*)-**P-1** (174.3 mg, 1.0 mmol) was dissolved in 5 mL of ethanol. The reaction flask was placed in an ice-water bath to cool the reaction solution to 0 °C, sodium borohydride (45.4 mg, 1.2 mmol) was slowly added into the reaction solution with stirring in batches, and the stirring was continued until the completion of reaction. The reaction was quenched by slow addition of 5% dilute hydrochloric acid. The reaction mixture was then extracted three times with ethyl acetate. The ethyl acetate phases were combined, washed with a small amount of saturated brine, dried with anhydrous sodium sulfate, concentrated under reduced pressure, and then quickly purified by silica gel column chromatography, to obtain 110 mg (S)-pantolactone with a yield of 85%, and 99% ee.

### Example 12

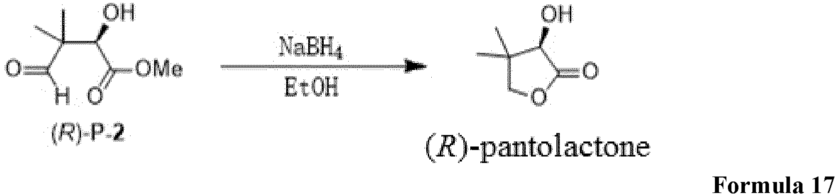

(*R*)-**P-2** (160.2 mg, 1.0 mmol) was dissolved in 5 mL of ethanol. The reaction flask was placed in an ice-water bath, and sodium borohydride (45.4 mg, 1.2 mmol) was slowly added into the reaction solution with stirring in batches, and the stirring was continued until the completion of reaction. The reaction was quenched by slow addition of 5% dilute hydrochloric acid. The reaction mixture was then extracted three times with ethyl acetate. The ethyl acetate phases were combined, washed with a small amount of saturated brine, dried with anhydrous sodium sulfate, concentrated under reduced pressure, and then quickly purified by silica gel column chromatography, to obtain 113 mg (*R*)-pantolactone with a yield of 87%, and 99% ee.

### Example 13

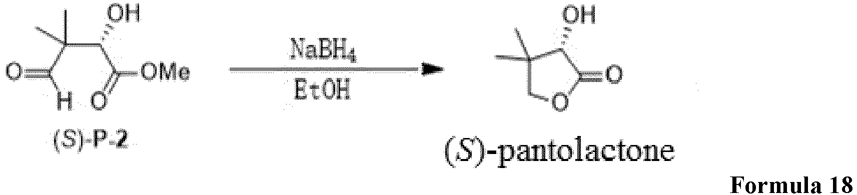

(*S*)-**P-2** (160.2 mg, 1.0 mmol) was dissolved in 5 mL of ethanol. The reaction flask was placed in an ice-water bath to cool the reaction solution to 0 °C, sodium borohydride (45.4 mg, 1.2 mmol) was slowly added into the reaction solution with stirring in batches, and the stirring was continued until the completion of reaction. The reaction was quenched by slow addition of 5% dilute hydrochloric acid. The reaction mixture was then extracted three times with ethyl acetate. The ethyl acetate phases were combined, washed with a small amount of saturated brine, dried with anhydrous sodium sulfate, concentrated under reduced pressure, and then quickly purified by silica gel column chromatography, to obtain 117 mg (S)-pantolactone with a yield of 90%, and 99% ee.

Characterizations of products **P-1 - P-6, Q-1 - Q-24** and pantolactone were as follows:

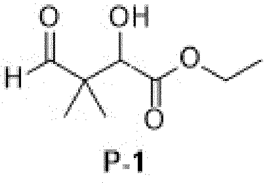

**P-1,** ¹H NMR (600 MHz, CDCl₃) δ 9.59 (s, 1H), 4.34 (d, *J =* 6.0 Hz, 1H), 4.27 (m, 2H), 3.02 (d, *J =* 5.4 Hz, 1H), 1.30 (t, *J =* 7.2 Hz, 3H), 1.16 (s, 3H), 1.08 (s, 3H); ¹³C NMR (100 MHz, CDCl₃) δ 202.7, 172.9, 77.1, 73.6, 62.4, 50.5, 18.3, 16.9, 14.2.

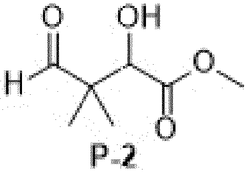

**P-2,** ¹H NMR (600 MHz, CDCl₃) δ 9.58 (s, 1H), 4.37 (d, *J =* 6.0 Hz, 1H), 3.81 (s, 3H), 2.96 (d, *J =* 6.0 Hz, 1H), 1.16 (s, 3H), 1.08 (s, 3H); ¹³C NMR (150 MHz, CDCl₃) δ 202.5, 173.3, 73.8, 52.7, 50.3, 18.2, 17.1.

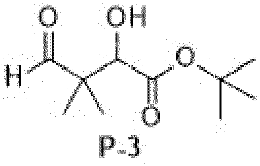

**P-3,** ¹H NMR (600 MHz, CDCl₃) δ 9.58 (s, 1H), 4.22 (d, *J =* 5.4 Hz, 1H), 3.05 (d, *J* = 5.4 Hz, 1H), 1.48 (s, 9H), 1.15 (s, 3H), 1.04 (s, 3H); ¹³C NMR (150 MHz, CDCl₃) δ 202.3, 172.0, 84.2, 73.3, 50.6, 27.9, 18.6, 16.0.

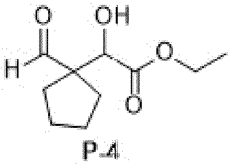

**P-4,** ¹H NMR (600 MHz, CDCl₃) δ 9.59 (s, 1H), 4.37 (d, *J =* 5.4 Hz, 1H), 4.26 (m, 2H), 3.11 (d, *J =* 5.4 Hz, 1H), 2.00 - 1.86 (m, 3H), 1.76 - 1.55 (m, 5H), 1.29 (t, *J =* 7.2 Hz, 3H); ¹³C NMR (150 MHz, CDCl₃) δ 201.6, 173.2, 72.4, 62.2, 61.6, 30.3, 28.1, 25.9, 25.7, 14.0.

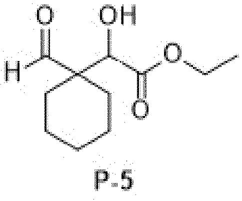

**P-5**, ¹H NMR (600 MHz, CDCl₃) δ 9.59 (s, 1H), 4.32-4.20 (m, 2H), 4.15 (d, *J =* 5.4 Hz, 1H), 2.97 (d, *J =* 4.8 Hz, 1H), 1.99-1.94 (m, 1H), 1.82-1.78 (m, 1H), 1.72 - 1.48 (m, 6H), 1.45-1.37 (m, 1H), 1.30 (t, *J =* 7.2 Hz, 3H), 1.26 - 1.14 (m, 2H); ¹³C NMR (150 MHz, CDCl₃) δ 204.5, 172.8, 77.2, 77.0, 76.8, 73.9, 62.2, 53.5, 27.8, 25.9, 25.1, 22.3, 22.1, 14.1.

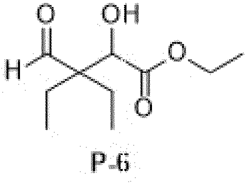

**P-6,** ¹H NMR (600 MHz, CDCl₃) δ 9.61 (s, 1H), 4.41 (s, 1H), 4.26 (m,2H), 2.97 (s, 1H), 1.79 - 1.63 (m, 4H), 1.29 (t, *J =* 7.2 Hz, 3H), 0.89 (td, *J =* 7.8, 3.0 Hz, 6H); ¹³C NMR (150 MHz, CDCl₃) δ 204.0, 173.5, 72.6, 62.2, 55.8, 22.6, 21.7, 14.0, 8.0, 7.90.

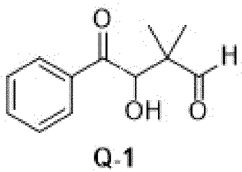

**Q-1,** ¹H NMR (400 MHz, CDCl₃) δ 9.62 (s, 1H), 7.88-7.86 (m, 2H), 7.66 - 7.62 (m, 1H), 7.51 (t, *J =* 8.0 Hz 2H), 5.28 (d, *J =* 8.0 Hz, 1H), 3.74 (d, *J =* 8.0 Hz, 1H), 1.09 (s, 3H), 0.96 (s, 3H); ¹³C NMR (100 MHz, CDCl₃) δ 203.8, 201.0, 136.0, 134.2, 129.2 129.0, 75.9, 50.1, 19.5, 17.5; HRMS: (ESI+) m/z calcd for [C₁₂H₁₄O₃ + H⁺] 207.1016, found 207.1019.

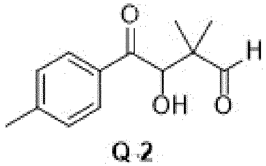

**Q-2,** ¹H NMR (600 MHz, CDCl₃) δ 9.62 (s, 1H), 7.77 (d, *J =* 8.0Hz, 2H), 7.30 (d, *J* =8.0 Hz, 2H), 5.25 (d, *J =* 8.0 Hz, 1H), 3.73 (d, *J =* 8.0 Hz, 1H), 2.43 (s, 3H), 1.08 (s, 3H), 0.95 (s, 3H); ¹³C NMR (150 MHz, CDCl₃) δ 203.8, 200.5, 145.4, 133.4, 129.6, 128.8, 75.7, 50.2, 21.8, 19.5, 17.5; HRMS: (ESI+) m/z calcd for [C₁₃H₁₆O₃ + H⁺] 221.1172, found 221.1172.

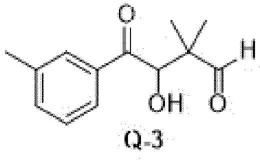

**Q-3,** ¹H NMR (400 MHz, CDCl₃) δ 9.55 (s, 1H), 7.62 - 7.54 (m, 2H), 7.37 (d, *J =* 7.2 Hz, 1H), 7.31 (t, *J =* 7.6 Hz, 1H), 5.20 (d, *J =* 8.0 Hz, 1H), 3.67 (d, *J =* 8.0 Hz, 1H), 2.36 (s, 3H), 1.01 (s, 3H), 0.87 (s, 3H); ¹³C NMR (100 MHz, CDCl₃) δ 202.7, 200.2, 137.9, 135.1, 134.0, 128.0, 127.7, 124.9, 74.9, 49.1, 20.3, 18.5, 16.5; HRMS: (ESI+) m/z calcd for [C₁₃H₁₆O₃ + H⁺] 221.1172, found 221.1175.

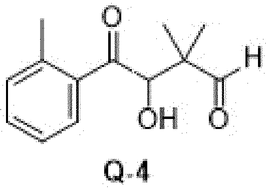

**Q-4,** ¹H NMR (400 MHz, CDCl₃) δ 9.51 (s, 1H), 7.53 (d, *J =* 8.0 Hz, 1H), 7.45-7.43 (m, 1H), 7.32-7.27 (m, 2H), 5.20 (d, *J =* 6.8 Hz, 1H), 3.88 (d, *J =* 7.2 Hz, 1H), 2.48 (s, 3H), 1.06 (s, 3H), 0.87 (s, 3H); ¹³C NMR (100 MHz, CDCl₃) δ 203.9, 202.8, 138.2, 135.9, 132.5, 132.3, 128.9, 125.7, 50.9, 20.5, 18.8, 17.9; HRMS: (ESI+) m/z calcd for [C₁₃H₁₆O₃ + H⁺] 221.1172, found 221.1174.

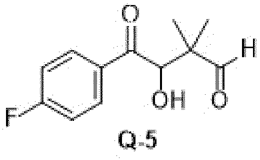

**Q-5,** ¹H NMR (400 MHz, CDCl₃) δ 9.57 (s, 1H), 7.94 - 7.82 (m, 2H), 7.11 (t, *J =* 8.4 Hz, 2H), 5.14 (s, 1H), 3.63 (s, 1H), 1.03 (s, 3H), 0.92 (s, 3H); ¹³C NMR (100 MHz, CDCl₃) δ 204.2, 199.1, 131.6, 131.5, 116.3, 116.1, 76.2, 49.8, 19.7, 17.8; HRMS: (ESI+) m/z calcd for [C₁₂H₁₃F₁O₃ + H⁺] 225.0921, found 225.0923.

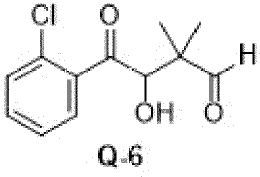

**Q-6,** ¹H NMR (400 MHz, CDCl₃) δ 9.51 (s, 1H), 7.49-7.46 (m, 2H), 7.45 (d, *J* = 2.0 Hz, 1H), 7.41-7.35 (m, 1H), 5.29 (d, *J =* 2.8 Hz, 1H), 3.73 (d, *J =* 6.8 Hz, 1H), 1.12 (s, 3H), 0.93 (s, 3H); ¹³C NMR (100 MHz, CDCl₃) δ 202.8, 202.6, 136.9, 133.0, 131.4, 131.1, 129.6, 127.1, 78.4, 50.9, 18.3, 17.8; HRMS: (ESI+) m/z calcd for [C₁₂H₁₃ClO₃ + H⁺] 241.0626, found 241.0629.

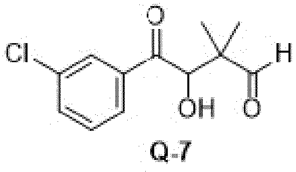

**Q-7,** ¹H NMR (400 MHz, CDCl₃) δ 9.64 (s, 1H), 7.88 (s, 1H), 7.77 (d, *J =* 8.8 Hz, 1H), 7.60 (d, *J =* 9.2 Hz, 1H), 7.49-7.43(m, 1H), 5.18 (s, 1H), 3.69 (d, *J* = 7.6 Hz, 1H), 1.11 (s, 3H), 1.00 (s, 3H); ¹³C NMR (100 MHz, CDCl₃) δ 204.1, 199.6, 137.5, 135.3, 134.0, 130.1, 128.7, 126.8, 76.5, 49.7, 19.7, 17.7; HRMS: (ESI+) m/z calcd for [C₁₂H₁₃ClO₃ + H⁺] 241.0626, found246.0629.

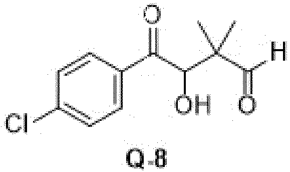

**Q-8,** ¹H NMR (400 MHz, CDCl₃) δ 9.63 (s, 1H), 7.84 (d, *J =* 8.8 Hz, 2H), 7.48 (d, *J* = 8.8 Hz, 2H), 5.20 (s, 1H), 3.70 (s, 1H), 1.10 (s, 3H), 1.00 (s, 3H); ¹³C NMR (100 MHz, CDCl₃) δ 204.2, 199.6, 140.8, 134.2, 130.2, 129.3, 76.3, 49.8, 19.7, 17.8; HRMS: (ESI+) m/z calcd for [C₁₂H₁₃ClO₃ + H⁺] 241.0626, found 241.0630.

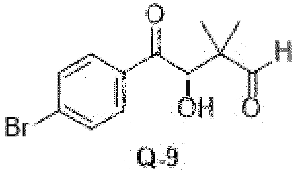

**Q-9,** ¹H NMR (600 MHz, CDCl₃) δ 9.63 (s, 1H), 7.76 (d, *J =* 9.0 Hz, 2H), 7.65 (d, *J* = 8.4Hz, 2H), 4.11 (s, 1H), 3.65 (d, *J =* 8.4 Hz, 1H), 1.10 (s, 3H), 1.00 (s, 3H); ¹³C NMR (150 MHz, CDCl₃) δ 204.1, 199.7, 134.7, 132.3, 130.2, 129.5, 76.3, 49.8, 19.7, 17.8; HRMS: (ESI+) m/z calcd for [C₁₂H₁₃BrO₃ + H⁺] 287.0100, found 287.0099.

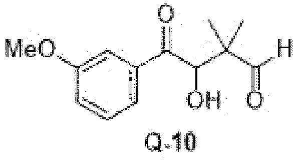

**Q-10,** ¹H NMR (400 MHz, CDCl₃) δ 9.55 (s, 1H), 7.38 - 7.33 (m, 2H), 7.31-7.29 (m, 1H), 7.13 - 7.07 (m, 1H), 5.18 (d, *J =* 8.0 Hz, 1H), 3.80 (s, 3H), 3.64 (d, *J =* 8.0 Hz, 1H), 1.01 (s, 3H), 0.89 (s, 3H); ¹³C NMR (100 MHz, CDCl₃) δ 202.7, 199.9, 158.9, 136.3, 128.9, 120.2, 119.5, 111.9, 76.3, 76.0, 75.7, 75.0, 54.5, 49.1, 18.5, 16.5; HRMS: (ESI+) m/z calcd for [C₁₃H₁₆O₄ + H⁺] 237.1121, found 237.1116.

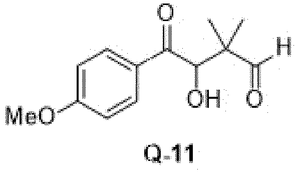

**Q-11,** ¹H NMR (600 MHz, CDCl₃) δ 9.64 (s, 1H), 7.89 - 7.86 (m, 2H), 6.98 - 6.95 (m, 2H), 5.23 (s, 1H), 3.89 (s, 3H), 1.08 (s, 3H), 0.98 (s, 3H). ¹³C NMR (150 MHz, CDCl₃) δ 204.1, 199.0, 164.5, 131.2, 128.6, 114.2, 7, 75.5, 55.6, 50.2, 19.6, 17.6; HRMS: (ESI+) m/z calcd for [C₁₃H₁₆O₄ + H⁺] 237.1121, found 237.1123.

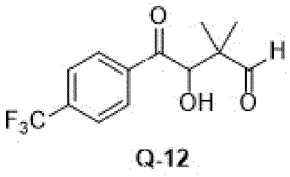

**Q-12,** ¹H NMR (600 MHz, CDCl₃) δ 9.64 (s, 1H), 8.01 (d, *J =* 8.4 Hz, 2H), 7.77 (d, *J =* 8.4 Hz, 2H), 5.21 (d, *J* = 7.8 Hz, 1H), 3.67 (d, *J =* 8.4 Hz, 1H), 1.13 (s, 3H), 1.02 (s, 3H); ¹³C NMR (150 MHz, CDCl₃) δ 204.2, 199.9, 138.8, 135.3, 135.1, 129.1, 125.9, 125.9, 49.6, 29.9, 19.8, 18.0; HRMS: (ESI+) m/z calcd for [C₁₃H₁₃F₃O₃ + H⁺] 297.0709, found 297.0703.

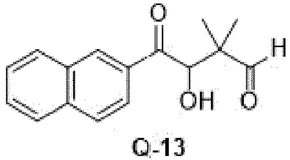

**Q-13,** ¹H NMR (400 MHz, CDCl₃) δ 9.67 (s, 1H), 8.41 (s, 1H), 8.03-7.85 (m, 4H), 7.68-7.55 (m, 2H), 5.44 (d, *J =* 8.0Hz, 1H), 3.79 (d, *J =* 8.0 Hz, 1H), 1.13 (s, 3H), 0.99 (s, 3H); ¹³C NMR (100 MHz, CDCl₃) δ 204.0, 200.8, 136.0, 133.2, 132.2, 130.8, 129.7, 129.2, 129.0, 127.9, 127.3, 123.9, 76.0, 50.1, 19.8, 17.6; HRMS: (ESI+) m/z calcd for [C₁₆H₁₆O₃ + H⁺] 257.1172, found 257.1173.

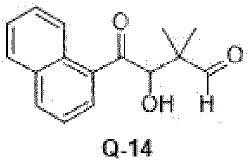

**Q-14,** ¹H NMR (600 MHz, CDCl₃) δ 9.51 (s, 1H), 8.38 (d, *J =* 8.4 Hz, 1H), 8.06 (d, *J= 8.4* Hz, 1H), 7.92 (d, *J =* 7.8 Hz, 1H), 7.82-7.79 (m, 1H), 7.6-7.49 (m, 3H), 5.37 (d, *J =* 6.6 Hz, 1H), 4.02 (d, *J =* 7.2 Hz, 1H), 1.10 (s, 3H), 0.84 (s, 3H); ¹³C NMR (150 MHz, CDCl₃) δ 203.8, 202.78, 134.0, 134.0, 129.6, 128.8, 128.6, 128.4, 127.0, 124.9, 124.1, 77.5, 51.1, 19.1, 17.6; HRMS: (ESI+) m/z calcd for [C₁₆H₁₆O₃ + H⁺] 257.1172, found 257.1179.

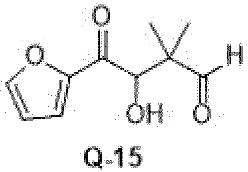

**Q-15,** ¹H NMR (400 MHz, CDCl₃) δ 9.67 (s, 1H), 7.68-7.67 (m, 1H), 7.39-7.37 (m, 1H), 6.63 (m, 1H), 5.07 (s, 1H), 3.49 (d, *J* = 7.6 Hz, 1H), 1.15 (s, 3H), 1.04 (s, 3H); ¹³C NMR (100 MHz, CDCl₃) δ 203.9, 188.4, 151.2, 147.7, 120.1, 113.2, 76.0, 50.7, 18.8, 17.5; HRMS: (ESI+) m/z calcd for [C₁₀H₁₂O₄ + H⁺] 197.0808, found 197.0810.

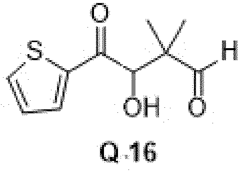

**Q-16,** ¹H NMR (400 MHz, CDCl₃) δ 9.59 (s, 1H), 7.76 (d, *J =* 4.0 Hz, 1H), 7.69 (d, *J* = 4.8 Hz, 1H), 7.12-7.10 (m, 1H), 4.94 (d, *J =* 6.4 Hz, 1H), 3.47 (d, *J* = 7.2 Hz, 1H), 1.10 (s, 3H), 1.02 (s, 3H); ¹³C NMR (100 MHz, CDCl₃) δ 203.3, 191.3, 140.8, 134.8, 133.3, 127.4, 48.9, 18.6, 16.6; HRMS: (ESI+) m/z calcd for [C₁₀H₁₂O₃S + H⁺] 213.0580, found 213.0581..

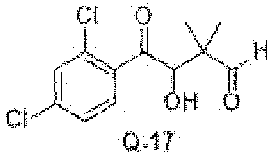

**Q-17,** ¹H NMR (600 MHz, CDCl₃) δ 9.52 (s, 1H), 7.49 (d, *J =* 1.8 Hz, 1H), 7.43 (d, *J =* 8.4 Hz, 1H), 7.38-7.35 (m, 1H), 5.22 (d, *J =* 6.0 Hz, 1H), 3.68 (d, *J =* 6.6 Hz, 1H), 1.13 (s, 3H), 0.97 (s, 3H); ¹³C NMR (150 MHz, CDCl₃) δ 202.9, 201.8, 138.7, 135.4, 132.5, 131.0, 130.6, 127.6, 78.6 , 50.8, 29.9, 22.5, 18.5, 18.0; HRMS: (ESI+) m/z calcd for [C₁₂H₁₂Cl₂O₃ + H⁺] 275.0236, found 275.0244..

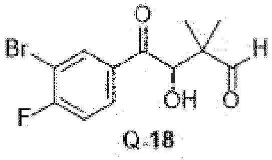

**Q-18,** ¹H NMR (600 MHz, CDCl₃) δ 9.65 (s, 1H), 8.17 (dd, *J* =6.6, *J =* 2.4 Hz, 1H), 7.90-7.86 (m, 1H), 7.24 (t, *J =* 8.4 Hz, 1H), 5.12 (s, 1H), 3.62 (s, 1H), 1.14 (s, 3H), 1.05 (s, _{3H}); ¹³C NMR (150 MHz, CDCl₃) δ 204.6, 197.7, 163.4, 161.7, 134.8, 134.8, 130.1 117.0, 116.8, 49.5, 19.8, 18.1; HRMS: (ESI+) m/z calcd for [C₁₂H₁₂Br₁F₁O₃ + H⁺] 305.0006, found 305.0011..

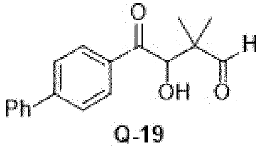

**Q-19,** ¹H NMR (600 MHz, CDCl₃) δ 9.66 (s, 1H), 7.98 - 7.94 (m, 2H), 7.75 - 7.70 (m, 2H), 7.66 - 7.61 (m, 2H), 7.50-7.46 (m, 2H), 7.45 - 7.40 (m, 1H), 5.30 (d, *J =* 8.4 Hz, 1H), 3.73 (d, *J =* 8.4 Hz, 1H), 1.12 (s, 3H), 1.01 (s, 3H). ¹³C NMR (150 MHz, CDCl₃) δ 204.0, 200.4, 147.0, 139.4, 134.6, 129.4, 129.0 , 128.6, 127.5,127.3, 76.0, 50.1, 19.7, 17.6; HRMS: (ESI+) m/z calcd for [C₁₈H₁₈O₃ + H⁺] 283.1329, found 283.1330..

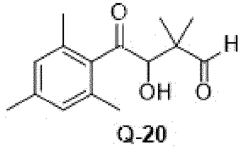

**Q-20,** ¹H NMR (600 MHz, CDCl₃) δ 9.47 (s, 1H), 6.86 (s, 2H), 4.83 (d, *J =* 6.6 Hz, 1H), 3.51 (d, *J =* 7.2 Hz, 1H), 2.27 (d, *J =* 11.4 Hz, 9H), 1.14 (s, 3H), 1.01 (s, 3H). ¹³C NMR (150 MHz, CDC₃) δ 208.7, 203.0, 140.3, 135.3, 134.9, 129.5, 80.8, 50.6, 21.1, 20.1, 18.8, 17.6; HRMS: (ESI+) m/z calcd for [C₁₅H₂₀O₃ + H⁺] 249.1485, found 249.1486..

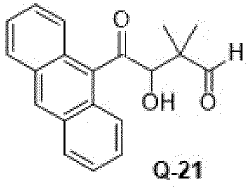

**Q-21,** ¹H NMR (600 MHz, CDCl₃) δ9.31 (s, 1H), 8.58 (s, 1H), 8.05 (d, *J =* 8.4 Hz, 2H), 7.96 (d, *J =* 9.0 Hz, 2H), 7.60 - 7.56 (m, 2H), 7.54 - 7.50 (m, 2H), 5.32 - 5.29 (m, 1H), 3.80 (d*, J =* 7.2 Hz, 1H), 1.10 (s, 3H), 0.74 (s, 3H). ¹³C NMR (150 MHz, CDCl₃) δ 208.6, 202.6, 131.8, 131.1, 131.0, 129.1, 128.5, 127.7, 125.7, 124.3, 81.8, 51.1, 17.9, 17.8; HRMS: (ESI+) m/z calcd for [C₂₀H₁₉O₃ + H⁺] 307.1329, found 307.1327.

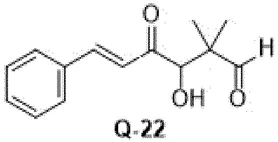

**Q-22,** ¹H NMR (600 MHz, CDCl₃) δ 9.68 (s, 1H), 7.76 (d, *J* = 15.6 Hz, 1H), 7.60-7.57 (m, 2H), 7.47-7.40 (m, 3H), 6.89 (d, *J =* 16.2 Hz, 1H), 4.64 (s, 1H), 3.63 (s, 1H), 1.16 (d, *J =* 1.2 Hz, 6H); ¹³C NMR (150 MHz, CDCl₃) δ 204.0, 198.7, 145.1, 133.9, 131.3, 129.1, 128.8, 121.8, 78.9, 50.5, 29.7, 18.7, 17.9; HRMS: (ESI+) m/z calcd for [C₁₄H₁₆O₃ + H⁺] 233.1172, found 233.1178..

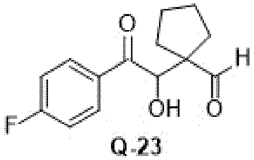

**Q-23,** ¹H NMR (600 MHz, CDCl₃) δ 9.71 (s, 1H), 8.15 - 7.93 (m, 2H), 7.21 - 7.12 (m, 1H), 5.04 (s, 1H), 3.93 (s, 1H), 2.12 - 2.02 (m, 1H), 1.97-1.1.84 (m, 2H), 1.72 - 1.55 (m, 4H), 1.26 (s, 1H).¹³C NMR (150 MHz, CDCl₃) δ 206.2, 198.0, 131.9,131.8, 116.0, 115.9, 78.0, 31.2, 30.9, 25.9, 25.4; HRMS: (ESI+) m/z calcd for [C₁₄H₁₅F₁O₃ + H⁺] 251.1078, found 251.1079..

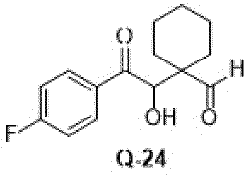

**Q-24,** ¹H NMR (600 MHz, CDCl₃) δ 9.70 (s, 1H), 7.96 - 7.90 (m, 2H), 7.20 - 7.15 (m, 2H), 5.00 (d, *J =* 6.0 Hz, 1H), 3.61 (d, *J =* 8.0 Hz, 1H), 2.04 - 1.99 (m, 1H), 1.65 - 1.56 (m, 4H), 1.40 - 1.24 (m, 5H); ¹³C NMR (150 MHz, CDCl₃) δ 207.2, 198.7, 167.1, 165.4, 131.7, 131.6, 116.2, 116.1, 77.8, 77.2, 77.0, 76.8, 53.0, 29.1, 28.3, 25.2, 22.5, 22.3; HRMS: (ESI+) m/z calcd for [C₁₅H₁₇O₃ F₁ + H⁺] 265.1234, found 265.1237..

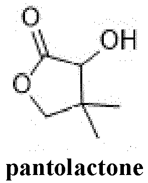

Pantolactone, ¹H NMR (400 M, CDCl₃) δ 4.14 (s, 1H), 4.04(d, *J*= 8.4Hz, 1H), 3.96 (d, *J*=9.2Hz, 1H), 2.95 (s, 1H), 1.24 (s, 3H), 1.09 (s, 3H).

The above description of the examples is only used to facilitate understanding of the method and core concept of the present disclosure.

## Claims

1. A method for synthesizing a chiral 2-hydroxy-1,4-dicarbonyl compound, comprising:
reacting a compound represented by Formula **(A)** with a compound represented by Formula **(B)** in the presence of a chiral catalyst with structure represented by Formula **(TP)** or Formula (***ent-*TP**)*,* to obtain a compound represented by Formula (*R*)-**P** or Formula (*S*)-**P**, 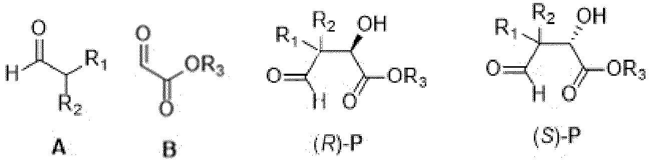 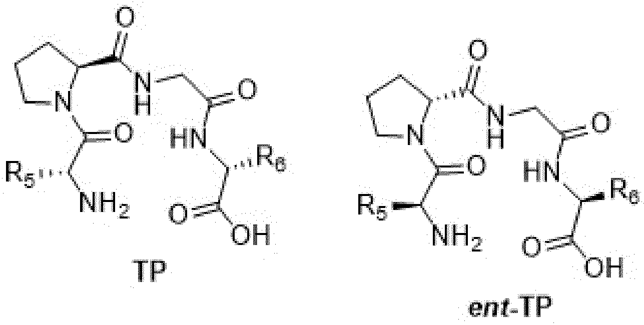
wherein R₁, R₂ are independently selected from straight-chain alkyl or branched-chain alkyl, or R₁ and R₂ together with the carbon to which they are bonded form cycloalkyl,
R₃ is selected from any one of C₁-C₄ straight-chain alkyl, branched-chain alkyl or benzyl, and
R₅, R₆ are independently selected from C₁-C₆ straight-chain alkyl, C₃-C₆ branched-chain alkyl, C₅-C₈ cycloalkyl, C₆-C₂₀ aryl or C₇-C₃₀ arylalkyl; or
reacting a compound represented by Formula **(A)** with a compound represented by Formula **(C)** in the presence of a catalyst with structure represented by Formula **(TP)** or Formula (***ent-*TP**)*,* to obtain a compound represented by Formula (*R*)-**Q** or Formula (*S*)-**Q**, 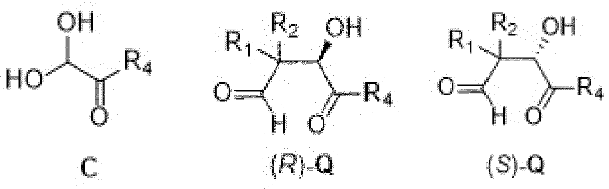
wherein R₄ is selected from any one of aryl, heteroaryl, arylethyl or arylvinyl.

2. The synthesis method according to claim 1, wherein R₅, R₆ are independently selected from methyl, ethyl, *n*-propyl, isopropyl, *n*-butyl, isobutyl, *tert*-butyl, *n*-pentyl, isopentyl,*n*-hexyl, cyclopentyl, cyclohexyl, cycloheptyl, phenyl, naphthyl, anthryl, benzyl or phenethyl.

3. The synthesis method according to claim 1, wherein the chiral catalyst is as follows

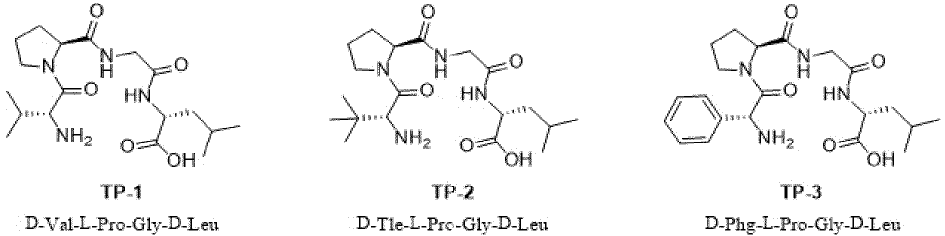

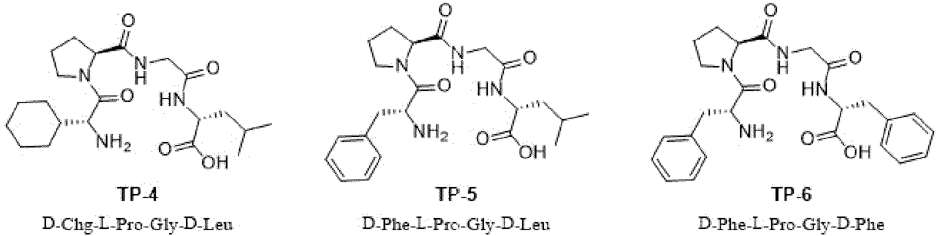

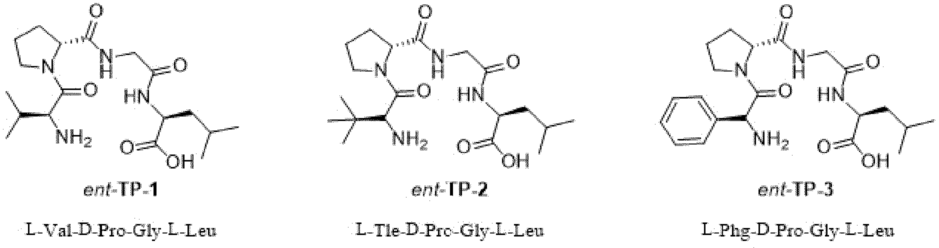

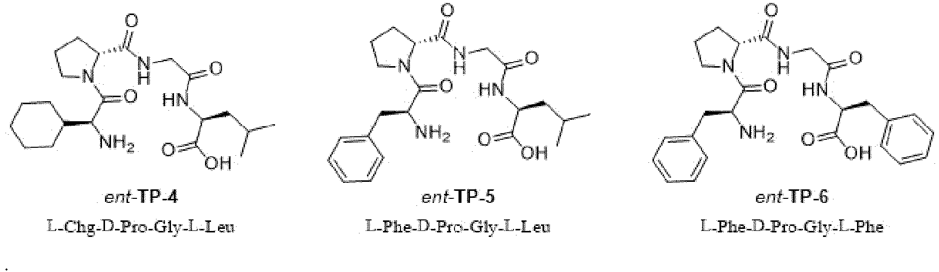

4. The synthesis method according to claim 1, wherein R₁, R₂ are independently selected from C₁-C₁₀ straight-chain alkyl or C₃-C₁₀ branched-chain alkyl, or R₁ and R₂ together with the carbon to which they are bonded form C₃-C₈ cycloalkyl;
R₃ is selected from C₁-C₄ straight-chain alkyl, or C₃-C₄ branched-chain alkyl; and
R₄ is phenyl, substituted phenyl, naphthyl, heteroaryl, anthryl or arylvinyl.

5. The synthesis method according to claim 1, wherein the method for synthesizing a chiral 2-hydroxy-1,4-dicarbonyl compound is a synthesis method by an asymmetric aldol reaction between aliphatic aldehyde and glyoxylate or between aliphatic aldehyde and acyl formaldehyde monohydrate by using tetrapeptide **TP** or its enantiomer ***ent*-TP** as a chiral catalyst, which is an asymmetric catalytic reaction having a chemical reaction equation as shown in Formula 1 or Formula 2: 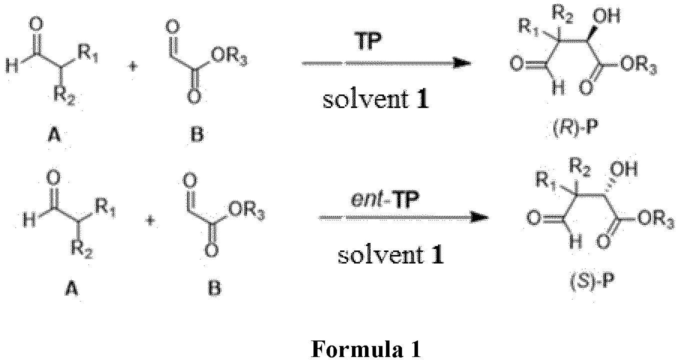 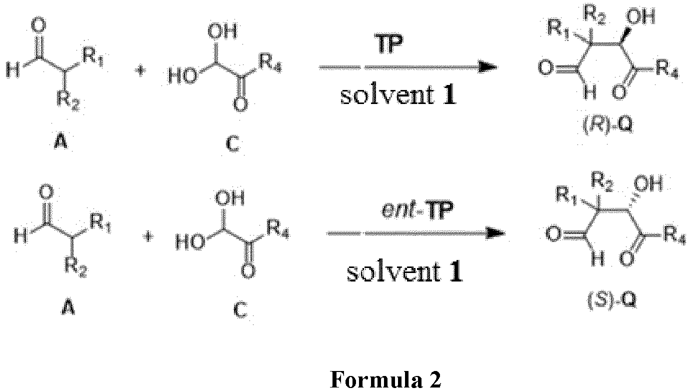
wherein the synthesis process comprises adding aliphatic aldehyde **A,** glyoxylate **B,** and tetrapeptide **TP** as shown in Formula 1 into a reaction vessel containing solvent **1** and stirring to obtain a product (*R*)-**P** in R configuration, or adding aliphatic aldehyde **A,** glyoxylate **B,** and tetrapeptide *ent-***TP** as shown in Formula 1 into a reaction vessel containing solvent **1** and stirring to obtain a product (*S*)-**P** in S configuration; or
adding aliphatic aldehyde **A,** acyl formaldehyde monohydrate **C**, and tetrapeptide **TP** as shown in Formula 2 into a reaction vessel containing solvent **1** and stirring to obtain a product (*R*)-**Q** in R configuration, or adding aliphatic aldehyde **A,** acyl formaldehyde monohydrate **C,** and tetrapeptide *ent-***TP** as shown in Formula 2 into a reaction vessel containing solvent **1** and stirring to obtain a product (*S*)-**Q** in S configuration;
wherein tetrapeptide **TP** or its enantiomer *ent-***TP** has structure as shown in Formula 3, 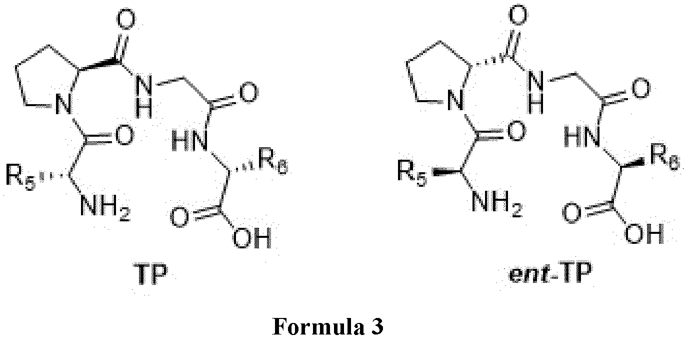
wherein R₁, R₂ are C₁-C₁₀ straight-chain alkyl or branched-chain alkyl, or R₁ and R₂ together with the carbon to which they are bonded form cycloalkyl;
R₃ is any one of C₁-C₄ straight-chain alkyl, branched-chain alkyl or benzyl;
R₄ is any one of aryl, heteroaryl, arylethyl or arylvinyl;
R₅, R₆ are any one of C₁-C₆ straight-chain alkyl, branched-chain alkyl, cyclohexyl, phenyl or benzyl; and
solvent **1** is any one of *n*-hexane, dichloromethane, chloroform, dichloroethane, diethyl ether, tetrahydrofuran, methyltetrahydrofuran, ethylene glycol dimethyl ether, dioxane, ethyl acetate, methyl acetate, ethyl formate, methyl formate, methyl *tert*-butyl ether, acetonitrile, propionitrile, butyronitrile, toluene, xylene, methanol, ethanol, isopropanol, or *n*-butanol, or a mixture thereof.

6. The synthesis method according to claim 5, wherein R₁, R₂ are independently selected from C₁-C₆ straight-chain alkyl, or R₁ and R₂ together with the carbon to which they are bonded form any one of cyclopropyl, cyclobutyl, cyclopentyl, cyclohexyl or cycloheptyl;
R₃ is selected from C₁-C₄ straight-chain alkyl or C₃-C₄ branched-chain alkyl;
R₄ is any one of phenyl, substituted phenyl, naphthyl, heteroaryl, anthryl or arylvinyl;
R₅ and R₆ are independently selected from any one of C₁-C₄ straight-chain alkyl, branched-chain alkyl, cyclohexyl, phenyl or benzyl; and
solvent **1** is any one of dichloromethane, 1,2-dichloroethane, chloroform, diethyl ether, tetrahydrofuran, ethyl acetate, acetonitrile, toluene, methanol, or methyl tert-butyl ether.

7. A method of preparing chiral pantolactone and an analogue thereof, comprising:
preparing the compound of Formula (*R*)-**P** or Formula (*S*)-**P** by using the method according to any one of claims 1-6 and performing a reduction reaction of the compound of formula (*R*)-**P** or Formula (*S*)-**P**, to obtain pantolactone in R or S configuration or an analogue thereof.

8. The preparation method according to claim 7, wherein the preparation method is carried out as shown in Formula 5, comprising adding (*R*)-**P** as shown in Formula 5 into a reaction vessel containing a reducing agent and solvent **2,** stirring and reacting to obtain (*R*)-pantolactone,
or adding (*S*)-**P** as shown in Formula 5 into a reaction vessel containing a reducing agent and solvent **2,** stirring and reacting to obtain (S)-pantolactone; wherein
R₄ is Me or Et,
solvent **2** is one or more of dichloromethane, 1,2-dichloroethane, tetrahydrofuran, ethyl acetate, methanol, ethanol and isopropanol; and
the reducing agent is one or more of borane, sodium cyanoborohydride, sodium triacetoxyborohydride, sodium borohydride, lithium borohydride and potassium borohydride;

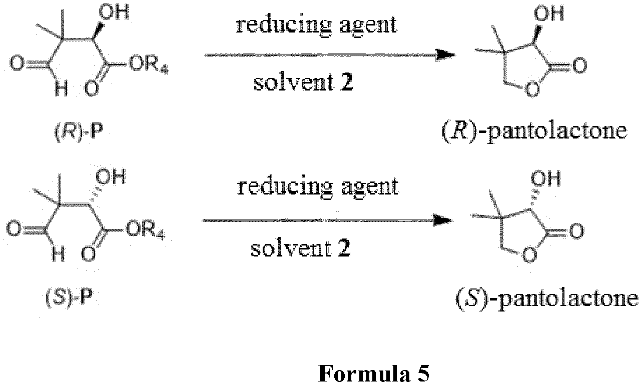

9. The preparation method according to claim 8, wherein solvent **2** is either methanol or ethanol; and
the reducing agent is any one of sodium cyanoborohydride, sodium triacetoxyborohydride or sodium borohydride.

## Patentansprüche

1. Verfahren zur Synthese einer chiralen 2-Hydroxy-1,4-dicarbonylverbindung, aufweisend:
Umsetzen einer durch Formel **(A)** dargestellten Verbindung mit einer durch Formel **(B)** dargestellten Verbindung in Gegenwart eines chiralen Katalysators mit einer durch Formel **(TP)** oder Formel **(*ent*-TP)** dargestellten Struktur, um eine durch Formel (*R*)**-P** oder Formel (*S*)-**P** dargestellte Verbindung zu erhalten, 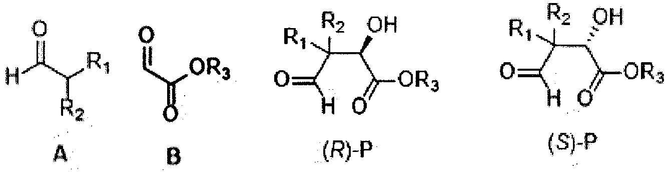 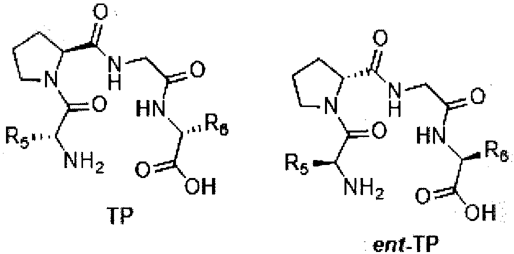
wobei R₁, R₂ unabhängig voneinander aus geradkettigem Alkyl oder verzweigtkettigem Alkyl ausgewählt sind, oder R₁ und R₂ zusammen mit dem Kohlenstoff, an den sie gebunden sind, Cycloalkyl bilden,
R₃ aus geradkettigem C₁-C₄-Alkyl, verzweigtkettigem Alkyl oder Benzyl ausgewählt ist, und
R₅, R₆ unabhängig voneinander aus geradkettigem C₁-C₆-Alkyl, verzweigtkettigem C₃-C₆-Alkyl, C₅-C₈-Cycloalkyl, C₆-C₂₀-Aryl oder C₇-C₃₀-Arylalkyl ausgewählt sind; oder
Umsetzen einer durch Formel **(A)** dargestellten Verbindung mit einer durch Formel **(C)** dargestellten Verbindung in Gegenwart eines Katalysators mit einer Struktur der Formel **(TP)** oder Formel **(*ent*-TP),** um eine durch Formel (*R*)-**Q** oder Formel (*S*)-**Q** dargestellte Verbindung zu erhalten, 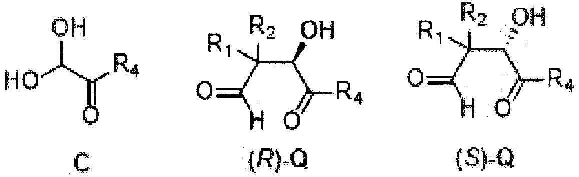
wobei R₄ aus einer der folgenden ausgewählt ist: Aryl, Heteroaryl, Arylethyl oder Arylvinyl.

2. Syntheseverfahren nach Anspruch 1, wobei R₅, R₆ unabhängig voneinander ausgewählt sind aus Methyl, Ethyl, *n*-Propyl, Isopropyl, *n*-Butyl, Isobutyl, *tert*-Butyl, *n-*Pentyl, Isopentyl, *n*-Hexyl, Cyclopentyl, Cyclohexyl, Cycloheptyl, Phenyl, Naphthyl, Anthryl, Benzyl oder Phenethyl.

3. Syntheseverfahren nach Anspruch 1, wobei der chirale Katalysator wie folgt ist:

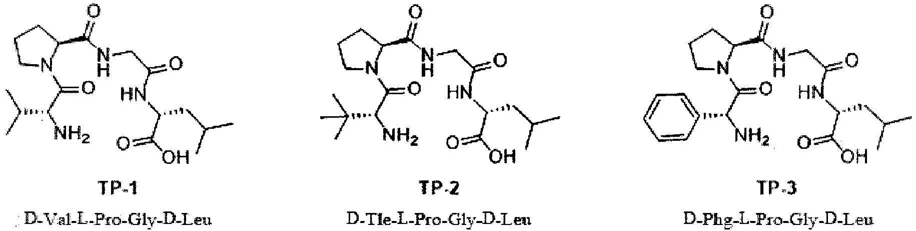

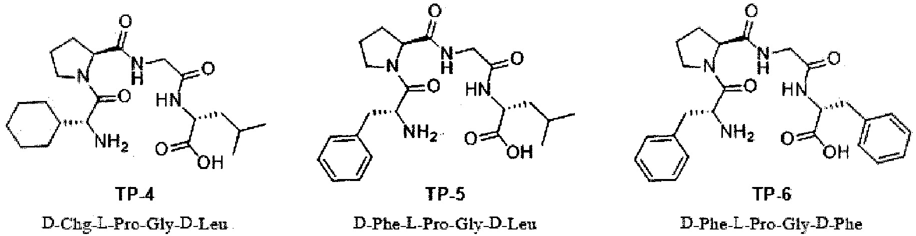

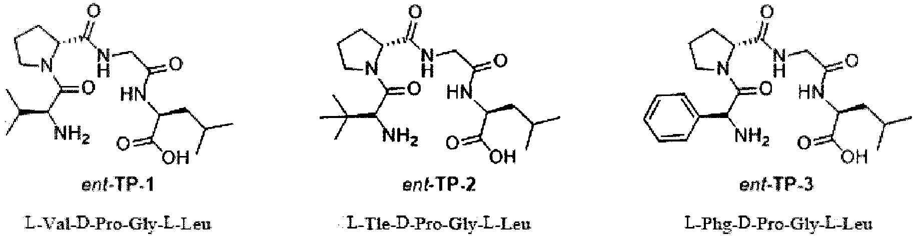

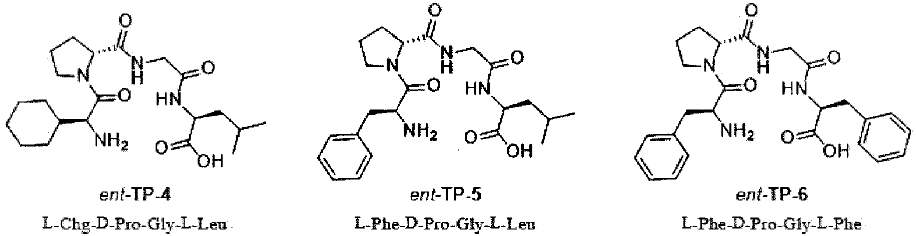

4. Syntheseverfahren nach Anspruch 1, wobei R₁, R₂ unabhängig voneinander ausgewählt sind aus geradkettigem C₁-C₁₀-Alkyl oder verzweigtkettigem C₃-C₄₀-Alkyl, oder R₁ und R₂ zusammen mit dem Kohlenstoff, an den sie gebunden sind, C₃-C₈-Cycloalkyl bilden;
R₃ aus geradkettigem C₁-C₄-Alkyl oder verzweigtkettigem C₃-C₄-Alkyl ausgewählt ist; und
R₄ Phenyl, substituiertes Phenyl, Naphthyl, Heteroaryl, Anthryl oder Arylvinyl ist.

5. Syntheseverfahren nach Anspruch 1, wobei das Verfahren zur Synthese einer chiralen 2-Hydroxy-1,4-dicarbonylverbindung ein Syntheseverfahren durch eine asymmetrische Aldolreaktion zwischen aliphatischem Aldehyd und Glyoxylat oder zwischen aliphatischem Aldehyd und Acylformaldehydmonohydrat ist, und zwar unter Verwendung von Tetrapeptid **TP** oder dessen Enantiomer *ent***-TP** als chiralen Katalysator, bei der es sich um eine asymmetrische katalytische Reaktion handelt, die eine chemische Reaktionsgleichung, wie in Formel 1 oder Formel 2 gezeigt, aufweist: 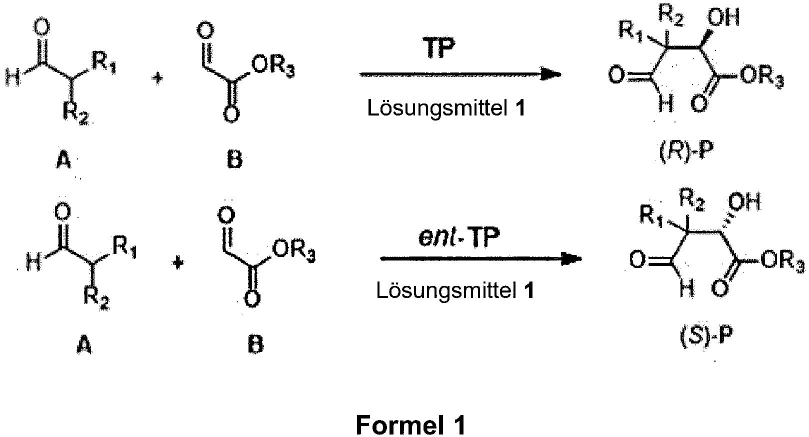 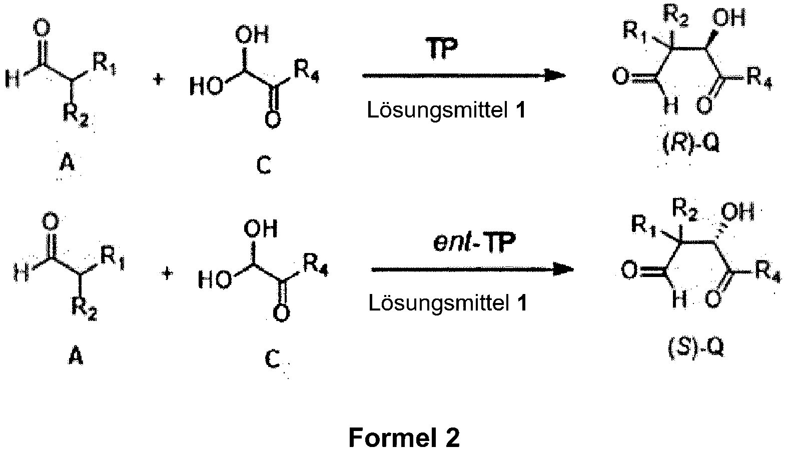
wobei der Synthesevorgang das Hinzufügen von aliphatischem Aldehyd **A,** Glyoxylat **B** und Tetrapeptid **TP,** wie ein Formel 1 gezeigt, in ein Reaktionsgefäß, das Lösungsmittel **1** enthält, und Rühren umfasst, um ein Produkt (*R*)-**P** in R-Konfiguration zu erhalten, oder das Hinzufügen von aliphatischem Aldehyd **A,** Glyoxylat **B,** und Tetrapeptid *ent-***TP,** wie in Formel 1 gezeigt, in ein Reaktionsgefäß, das Lösungsmittel **1** enthält, und Rühren umfasst, um ein Produkt (*S*)-**P** in S-Konfiguration zu erhalten; oder
das Hinzufügen von aliphatischem Aldehyd **A,** Acylformaldehydmonohydrat **C** und Tetrapeptid **TP,** wie in Formel 2 gezeigt, in ein Reaktionsgefäß, das Lösungsmittel **1** aufweist, und Rühren umfasst, um ein Produkt (*R*)-**Q** in R-Konfiguration erhalten, oder das Hinzufügen von aliphatischem Aldehyd **A,** Acylformaldehydmonohydrat **C** und Tetrapeptid *ent***-TP**, wie in Formel 2 gezeigt, in ein Reaktionsgefäß, das Lösungsmittel **1** aufweist, und Rühren umfasst, um ein Produkt (*S*)-**Q** in S-Konfiguration zu erhalten;
wobei das Tetrapeptid **TP** oder sein Enantiomer *ent*-**TP** die in Formel 3 gezeigte Struktur aufweist, 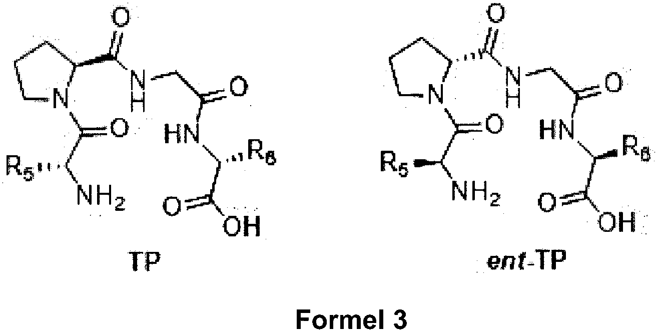
wobei R₁, R₂ geradkettiges C₁-C₁₀-Alkyl oder verzweigtkettiges Alkyl sind, oder R₁ und R₂ zusammen mit dem Kohlenstoff, an den sie gebunden sind, Cycloalkyl bilden;
R₃ eines von einem geradkettigem C₁-C₄-Alkyl, verzweigtkettigem Alkyl oder Benzyl ist;
R₄ eines von Aryl, Heteroaryl, Arylethyl oder Arylvinyl ist;
R₅, R₆ eines aus geradkettigem C₁-C₆-Alkyl, verzweigtkettigem Alkyl, Cyclohexyl, Phenyl oder Benzyl sind; und
Lösungsmittel **1** eines von *n*-Hexan, Dichlormethan, Chloroform, Dichlorethan,
Diethylether, Tetrahydrofuran, Methyltetrahydrofuran, Ethylenglykoldimethylether, Dioxan, Ethylacetat, Methylacetat, Ethylformiat, Methylformiat, Methyl-*tert*-butylether, Acetonitril, Propionitril, Butyronitril, Toluol, Xylol, Methanol, Ethanol, Isopropanol oder *n*-Butanol ist, oder eine Mischung davon.

6. Syntheseverfahren nach Anspruch 5, wobei R₁, R₂ unabhängig voneinander aus geradkettigem C₁-C₆-Alkyl ausgewählt sind, oder R₁ und R₂ zusammen mit dem Kohlenstoff, an den sie gebunden sind, eines der folgenden bilden: Cyclopropyl, Cyclobutyl, Cyclopentyl, Cyclohexyl oder Cycloheptyl;
R₃ aus geradkettigem C₁-C₄-Alkyl, oder verzweigtkettigem C₃-C₄-Alkyl ausgewählt ist;
R₄ eines von Phenyl, substituiertes Phenyl, Naphthyl, Heteroaryl, Anthryl oder Arylvinyl ist;
R₅ und R₆ unabhängig voneinander aus geradkettigem C₁-C₄-Alkyl, verzweigtkettigem Alkyl, Cyclohexyl, Phenyl oder Benzyl ausgewählt sind; und
Lösungsmittel **1** eines von Dichlormethan, 1,2-Dichlorethan, Chloroform, Diethylether, Tetrahydrofuran, Ethylacetat, Acetonitril, Toluol, Methanol oder Methyl-tert-butylether ist.

7. Verfahren zur Herstellung von chiralem Pantolacton und einem Analogon davon, aufweisend:
Herstellen der Verbindung der Formel (*R*)-**P** oder Formel (*S*)-**P** unter Einsatz des Verfahrens nach einem der Ansprüche 1 bis 6, und Durchführen einer Reduktionsreaktion der Verbindung der Formel (*R*)-**P** oder Formel (*S*)-**P**, um Pantolacton in R- oder S-Konfiguration oder ein Analogon davon zu erhalten.

8. Verfahren zur Herstellung nach Anspruch 7, wobei das Verfahren zur Herstellung wie in Formel 5 gezeigt durchgeführt wird, aufweisend das Hinzufügen von (*R*)-**P**, wie in Formel 5 gezeigt, in ein Reaktionsgefäß, das ein Reduktionsmittel und Lösungsmittel **2** enthält, Rühren und Umsetzen, um (*R*)-Pantolacton zu erhalten,
oder aufweisend das Hinzufügen von (*S*)-**P**, wie in Formel 5 gezeigt, in ein Reaktionsgefäß, das ein Reduktionsmittel und Lösungsmittel **2** enthält, Rühren und Umsetzen, um (*S*)-Pantolacton zu erhalten; wobei
R₄ Me oder Et ist,
Lösungsmittel **2** eines oder mehrere von Dichlormethan, 1,2-Dichlorethan, Tetrahydrofuran, Ethylacetat, Methanol, Ethanol und Isopropanol ist; und
das Reduktionsmittel eines oder mehrere von Boran, Natriumcyanoborhydrid, Natriumtriacetoxyborhydrid, Natriumborhydrid, Lithiumborhydrid und Kaliumborhydrid ist;

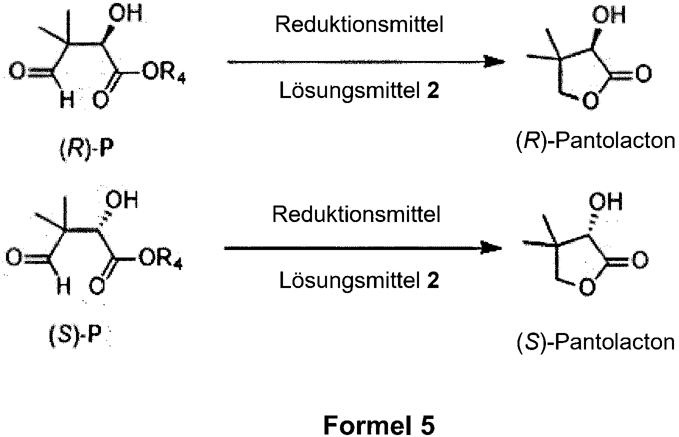

9. Verfahren nach Anspruch 8, wobei Lösungsmittel **2** entweder Methanol oder Ethanol ist; und
das Reduktionsmittel eines von Natriumcyanoborhydrid, Natriumtriacetoxyborhydrid oder Natriumborhydrid ist.

## Revendications

1. Procédé pour synthétiser un composé 2-hydroxy-1,4-dicarbonyle chiral, comportant de :
faire réagir un composé représenté par la Formule (A) avec un composé représenté par la Formule (B) en présence d'un catalyseur chiral ayant la structure représentée par la Formule (TP) ou la Formule (ent-TP), pour obtenir un composé représenté par la Formule (R)-P ou la Formule (*S*)-**P**, 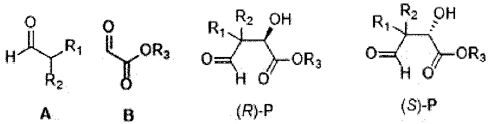 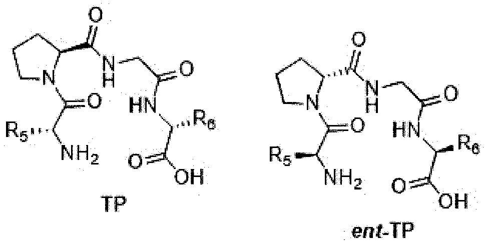
dans lesquelles R₁ et R₂ sont choisis indépendamment les uns des autres parmi un alkyle à chaîne droite ou un alkyle à chaîne ramifiée, ou R₁ et R₂ forment, en association avec le carbone auquel ils sont rattachés, un cycloalkyle,
R₃ est choisi parmi un élément quelconque parmi un alkyle à chaîne droite en C₁ à C₄, un alkyle à chaîne ramifiée ou un benzyle, et
R₅, R₆ sont choisis indépendamment les uns des autres parmi un alkyle à chaîne droite en C₁ à C₆, un alkyle à chaîne ramifiée en C₃ à C₆, un cycloalkyle en C₅ à C₈, un aryle en C₆ à C₂₀ ou un arylalkyle en C₇ à C₃₀, ou
faire réagir un composé représenté par la Formule (A) avec un composé représenté par la Formule (C) en présence d'un catalyseur ayant la structure représentée par la Formule (TP) ou la Formule (ent-TP), pour obtenir un composé représenté par la Formule (R)-Q ou la Formule (S)-Q, 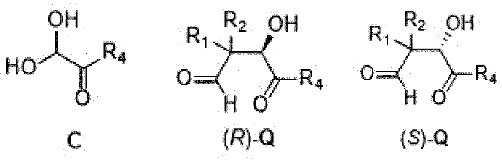
dans lesquelles R₄ est choisi parmi un élément quelconque parmi un aryle, un hétéroaryle, un aryléthyle ou un arylvinyle.

2. Procédé de synthèse selon la revendication 1, dans lequel R₅, R₆ sont choisis indépendamment les uns des autres parmi un méthyle, un éthyle, un n-propyle, un isopropyle, un *n*-butyle, un isobutyle, un *tert*-butyle*,* un *n*-pentyle, un isopentyle, un *n-*hexyle, un cyclopentyle, un cyclohexyle, un cycloheptyle, un phényle, un naphtyle, un anthryle, un benzyle ou un phénéthyle.

3. Procédé de synthèse selon la revendication 1, dans lequel le catalyseur chiral est comme suit :

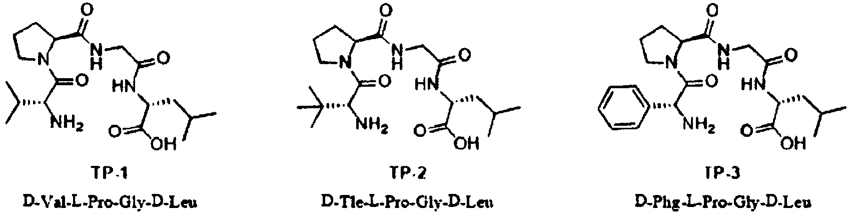

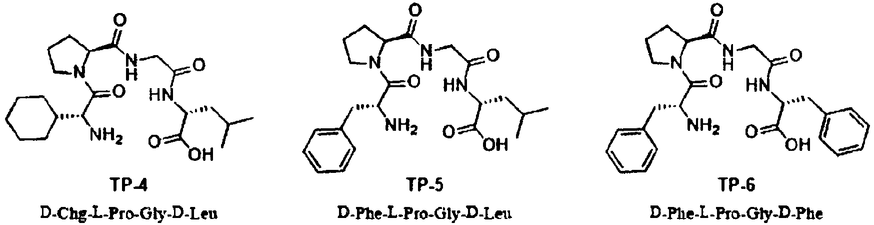

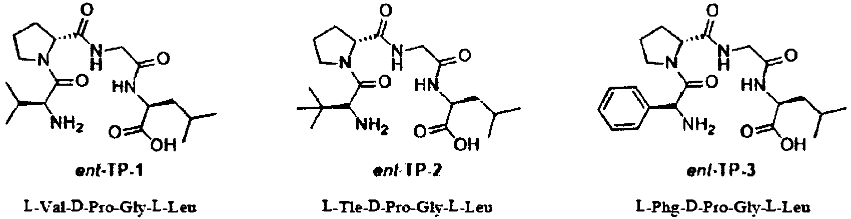

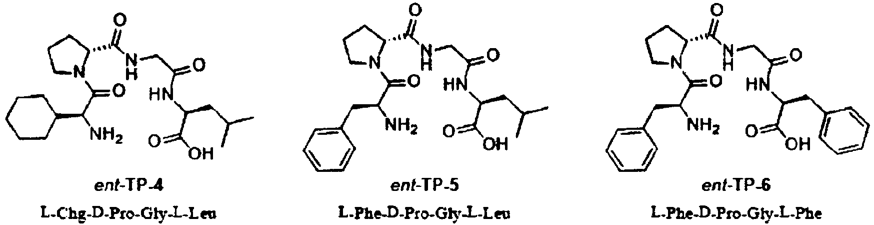

4. Procédé de synthèse selon la revendication 1, dans lequel R₁, R₂ sont choisis indépendamment les uns des autres parmi un alkyle à chaîne droite en C₄ à C₁₀ ou un alkyle à chaîne ramifiée en C₃ à C₁0, ou R₁ et R₂ forment, en association avec le carbone auquel ils sont rattachés, un cycloalkyle en C₃ à C₈,
R₃ est choisi parmi un alkyle à chaîne droite en C₁ à C₄ ou un alkyle à chaîne ramifiée en C₃ à C₄, et
R₄ est un phényle, un phényle substitué, un naphtyle, un hétéroaryle, un anthryle ou un arylvinyle.

5. Procédé de synthèse selon la revendication 1, dans lequel le procédé pour synthétiser un composé 2-hydroxy-1,4-dicarbonyle chiral est un procédé de synthèse par une réaction aldolique asymétrique entre un aldéhyde aliphatique et un glyoxylate ou entre un aldéhyde aliphatique et un acyl formaldéhyde monohydraté en utilisant un tétrapeptide TP ou son énantiomère ent-TP comme catalyseur chiral, qui est une réaction catalytique asymétrique ayant une équation de réaction chimique comme indiqué dans la Formule 1 ou la Formule 2 : 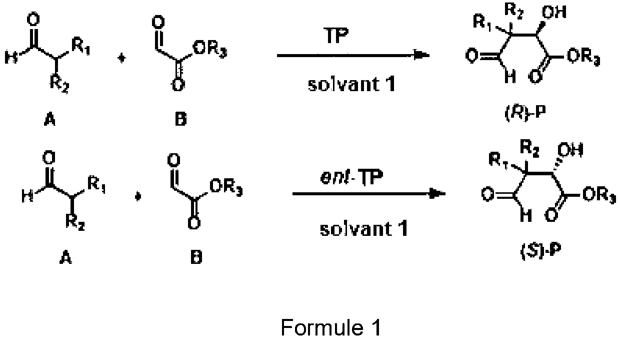 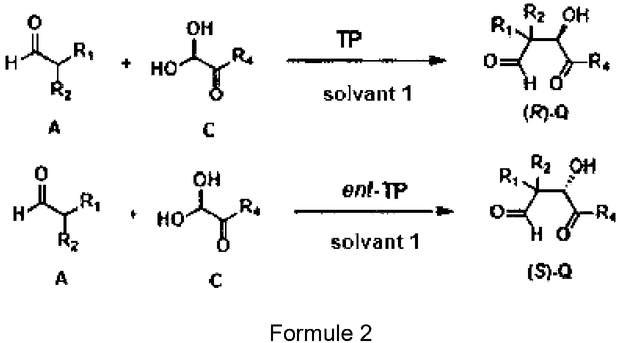
dans lequel le procédé de synthèse comporte les étapes consistant à ajouter un aldéhyde aliphatique A, un glyoxylate B et un tétrapeptide TP comme indiqué dans la Formule 1 dans un récipient de réaction contenant du solvant 1 et agiter pour obtenir un produit (R)-P en configuration R, ou ajouter l'aldéhyde aliphatique A, le glyoxylate B et le tétrapeptide ent-TP comme indiqué dans la Formule 1 dans un récipient de réaction contenant du solvant 1 et agiter pour obtenir un produit (S)-P en configuration S, ou
ajouter l'aldéhyde aliphatique A, l'acyl formaldéhyde monohydraté C et le tétrapeptide TP comme indiqué dans la Formule 2 dans un récipient de réaction contenant du solvant 1 et agiter pour obtenir un produit (R)-Q en configuration R, ou ajouter l'aldéhyde aliphatique A, l'acyl formaldéhyde monohydraté C et le tétrapeptide ent-TP comme indiqué dans la Formule 2 dans un récipient de réaction contenant du solvant 1 et agiter pour obtenir un produit (S)-Q en configuration S,
dans lequel le tétrapeptide TP ou son énantiomère ent-TP a la structure comme indiqué dans la Formule 3, 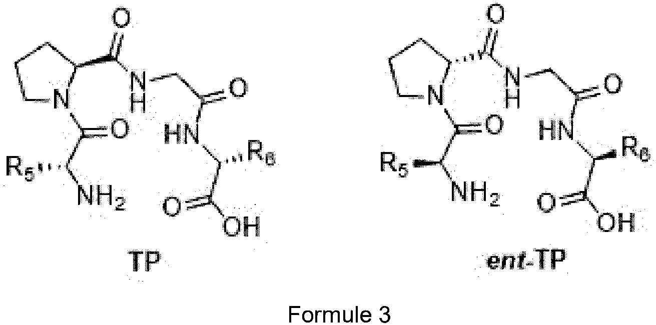
dans laquelle R₁ et R₂ sont un alkyle à chaîne droite ou un alkyle à chaîne ramifiée en C₁ à C₁₀, ou R₁ et R₂ forment, en association avec le carbone auquel ils sont rattachés, un cycloalkyle,
R₃ est un élément quelconque parmi un alkyle à chaîne droite en C₁ à C₄, un alkyle à chaîne ramifiée ou un benzyle,
R₄ est un élément quelconque parmi un aryle, un hétéroaryle, un aryléthyle ou un arylvinyle,
R₅, R₆ sont un élément quelconque parmi un alkyle à chaîne droite en C₁ à C₆, un alkyle à chaîne ramifiée, un cyclohexyle, un phényle ou un benzyle, et
le solvant 1 est un élément quelconque parmi : n-hexane, dichlorométhane, chloroforme, dichloroéthane, éther diéthylique, tétrahydrofurane, méthyltétrahydrofurane, éther diméthylique d'éthylène glycol, dioxane, acétate d'éthyle, acétate de méthyle, formiate d'éthyle, formiate de méthyle, éther méthyl-tert-butylique, acétonitrile, propionitrile, butyronitrile, toluène, xylène, méthanol, éthanol, isopropanol ou n-butanol, ou un mélange de ceux-ci.

6. Procédé de synthèse selon la revendication 5, dans lequel R₁, R₂ sont choisis indépendamment les uns des autres parmi un alkyle à chaîne droite en C₁ à C₆, ou R₁ et R₂ forment, conjointement avec le carbone auquel ils sont rattachés un élément quelconque parmi un cyclopropyle, un cyclobutyle, un cyclopentyle, un cyclohexyle ou un cycloheptyle,
R₃ est choisi parmi un alkyle à chaîne droite en C₁ à C₄ ou un alkyle à chaîne ramifiée en C₃ à C₄,
R₄ est un élément quelconque parmi un phényle, un phényle substitué, un naphtyle, un hétéroaryle, un anthryle ou un arylvinyle,
R₅ et R₆ sont choisis indépendamment les uns des autres parmi un élément quelconque parmi un alkyle à chaîne droite en C₁ à C₄, un alkyle à chaîne ramifiée, un cyclohexyle, un phényle ou un benzyle , et
le solvant 1 est un élément quelconque parmi : dichlorométhane, 1,2-dichloroéthane, chloroforme, éther diéthylique, tétrahydrofurane, acétate d'éthyle, acétonitrile, toluène, méthanol ou éther méthyl tert-butylique.

7. Procédé de préparation de pantolactone chirale et d'un analogue de celle-ci, comportant de :
préparer le composé de Formule (R)-P ou de Formule (S)-P en utilisant le procédé selon l'une quelconque des revendications 1 à 6 et réaliser une réaction de réduction du composé de Formule (R)-P ou de Formule (S)-P, pour obtenir de la pantolactone en configuration R ou S ou un analogue de celle-ci.

8. Procédé de préparation selon la revendication 7, dans lequel le procédé de préparation est mis en œuvre comme indiqué dans la Formule 5, comportant les étapes consistant à ajouter (R)-P comme indiqué dans la Formule 5 dans un récipient de réaction contenant un agent réducteur et du solvant 2, agiter et faire réagir pour obtenir de la (R)-pantolactone,
ou ajouter (S)-P comme indiqué dans la Formule 5 dans un récipient de réaction contenant un agent réducteur et du solvant 2, agiter et faire réagir pour obtenir de la (S)-pantolactone, dans lequel
R₄ est Me ou Et,
le solvant 2 est un ou plusieurs éléments parmi : dichlorométhane, 1,2-dichloroéthane, tétrahydrofurane, acétate d'éthyle, méthanol, éthanol et isopropanol, et
l'agent réducteur est un ou plusieurs éléments parmi : borane, cyanoborohydrure de sodium, triacétoxyborohydrure de sodium, borohydrure de sodium, borohydrure de lithium et borohydrure de potassium,

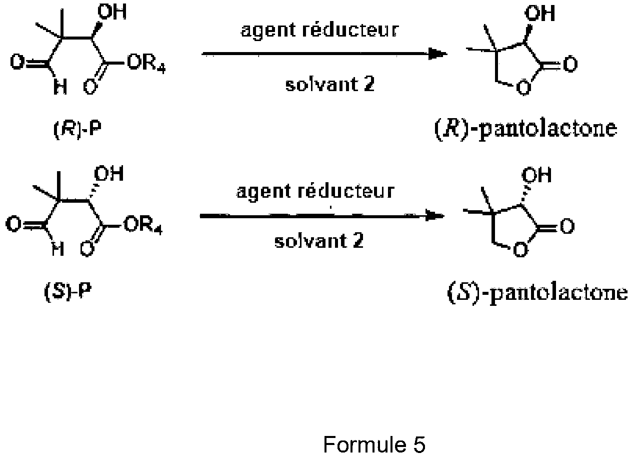

9. Procédé de préparation selon la revendication 8, dans lequel le solvant 2 est le méthanol ou l'éthanol, et
l'agent réducteur est un élément quelconque parmi : cyanoborohydrure de sodium, triacétoxyborohydrure de sodium ou borohydrure de sodium.
